(19) Europäisches Patentamt
European Patent Office
Office européen des brevets

(11) Veröffentlichungsnummer: **0 298 029 B1**

(12) **EUROPÄISCHE PATENTSCHRIFT**

(45) Veröffentlichungstag der Patentschrift: **02.12.92**

(51) Int. Cl.5: **C07D 401/04**, C07D 233/70, C07D 405/04, C07D 409/04, C07D 471/14, C07D 491/147, C07D 495/16, C07D 487/04, C07C 233/00

(21) Anmeldenummer: **88810424.7**

(22) Anmeldetag: **21.06.88**

Die Akte enthält technische Angaben, die nach dem Eingang der Anmeldung eingereicht wurden und die nicht in dieser Patentschrift enthalten sind.

# (54) Substituierte Imidazolone mit herbizider Wirkung.

(30) Priorität: **01.07.87 CH 2480/87**

(43) Veröffentlichungstag der Anmeldung:
**04.01.89 Patentblatt 89/01**

(45) Bekanntmachung des Hinweises auf die Patenterteilung:
**02.12.92 Patentblatt 92/49**

(84) Benannte Vertragsstaaten:
**AT BE CH DE ES FR GB GR IT LI LU NL SE**

(56) Entgegenhaltungen:
**EP-A- 0 041 623**
**EP-A- 0 041 624**
**EP-A- 0 133 309**
**CH-A- 639 957**
**DE-A- 2 700 270**
**DE-A- 3 345 901**
**DE-A- 3 403 730**
**DE-A- 3 420 271**
**FR-A- 2 081 505**
**GB-A- 2 172 886**
**US-A- 4 474 962**

(73) Patentinhaber: **CIBA-GEIGY AG**
**Klybeckstrasse 141**
**CH-4002 Basel(CH)**

(72) Erfinder: **Dingwall, John Grey, Dr.**
**St. Pantaleonstrasse 16**
**CH-4412 Nuglar(CH)**

EP 0 298 029 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

CHEMICAL ABSTRACTS, Band 105, Nr. 23, 8. Dezember 1986, Columbus, Ohio, US; NUMATA, TATSUO et al.: "Imidazolylthienopyridine derivatives as herbicides." Seite 597, Spalte 1, Zusammenfassung-Nr. 208 886n

CHEMICAL ABSTRACTS, Band 103, Nr. 3, 22. Juli 1985, Columbus, Ohio, US; NISSAN CHEMICAL INDUTRIES, LTD.: "Substituted nicotinic acid derivatives." Seite 578, Spalte 1, Zusammenfassung-Nr. 22 590q

CHEMICAL ABSTRACTS, Band 103, Nr. 3, 22. Juli 1985, Columbus, Ohio, US; NISSAN CHEMICAL INDUSTRIES, LTD.: "Substituted quinoline derivatives." Seite 578, Spalte 2, Zusammenfassung-Nr. 22 591r

CHEMICAL ABSTRACTS, Band 66, Nr. 21, 22. Mai 1967, Columbus, Ohio, US; L. KNUNYANTS et al.: "Derivatives of perfluoroisobutyricacid." Seite 8846, Spalte 1, Zusammenfassung-Nr. 94 675m

CHEMICAL Abstracts, Band 73, Nr. 1, 6. Juli 1970, Columbus, Ohio, US; MIDDLETON, WILLIAM J. et al.: "Fluorinated aminoimidazolines. Synthesis and determination of tautomeric structure." Seite 381, Spalte 2, Zusammenfassung-Nr. 3 856b

## Beschreibung

Die vorliegende Erfindung betrifft neue substituierte Imidazolone mit herbizider Wirkung, Verfahren zu deren Herstellung, Mittel, welche diese Verbindungen enthalten, neue Zwischenprodukte zu deren Herstellung sowie die Verwendung der erfindungsgemässen Verbindungen zur Bekämpfung von Unkräutern und Gräsern.

Aus der europäischen Patentanmeldung EP-A-0 041 623, der GB-A-2 172 886, der CH-B-639 957 und der DE-A-3 420 271 sind herbizid wirksame 1-[4,4-di-Alkyl-imidazol-1(H)5-on-2-yl]-benzol-2-carbonsäuren und 2-[4,4-di-Alkyl-imidazol-1(H)5-on-2-yl] pyridin-3-carbonsäuren, deren Ester, Imide und deren benzo- oder hetero-anellierten Derivate bekannt geworden. US 4,474,962 betrifft ein neues Verfahren zur Herstellung bestimmter 2-[4,4-di-alkyl-imidazol-1(H)5-on-2-yl]-pyridin-3-carbonsäuren. Es wurde nun gefunden, dass bestimmte, neue, 1-[4-Alkyl-4-fluor-$C_1$-$C_2$-alkyl-imidazol-1(H)5-on-2-yl]benzol-2-carbonsäuren und 2-[4-Alkyl-4-fluor-$C_1$-$C_2$-alkyl-imidazol-1(H)5-on-2-yl]-pyridin -3carbonsäuren der Formel I ebenfalls herbizid wirksam sind.

Die Erfindung umfasst die neuen Verbindungen der Formel I

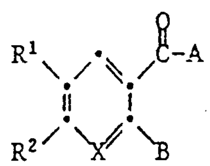

I,

worin

- X CH oder N
- $R^1$ und $R^2$ unabhängig voneinander Wasserstoff; ein gegebenenfalls ein- oder mehrfach durch Halogen, Nitro, Cyano, $C_1$-$C_4$-Alkoxy, $C_1$-$C_4$-Halogenalkoxy, $C_1$-$C_4$-Alkylthio oder $C_1$-$C_4$-Halogenalkylthio substituiertes $C_1$-$C_4$-Alkyl; oder
- $R^1$ und $R^2$ gemeinsam mit den Kohlenstoffatomen des Sechsringes, an welchen sie gebunden sind einen ungesättigten oder teilweise ungesättigten carbocyclischen oder bis zu zwei O oder S-Atome enthaltenden heterocyclischen 5 oder 6-Ring bilden; und
- A OH; $O^-M^+$; $C_1$-$C_4$-Alkoxy; $C_3$-$C_5$-Alkenyloxy; $C_3$-$C_5$-Alkinyloxy oder $C_1$-$C_4$-Alkoxy-$C_1$-$C_4$-alkoxy; und
- $M^+$ ein Kationenäquivalent eines Alkali- oder Erdalkalymetalls oder einer stickstoffhaltigen Base; und
- B ein Imidazolon der Formel

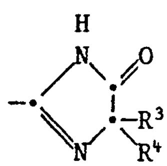

bilden; und

- $R^3$ $C_1$-$C_4$-Alkyl; und
- $R^4$ ein mit 1 bis 5 Fluoratomen substituierten Methyl- oder Ethylrest bedeutet,

sowie Salze von Verbindungen der Formel I mit Säuren, Basen und Komplexbildnern.

Die als mögliche Substituenten der Formel I angegebenen generischen Begriffe umfassen beispielsweise die nachstehend angegebenen spezifischen Einzelsubstituenten.

Halogen ist Fluor, Chlor, Brom und Jod; in erster Linie Fluor, Chlor und Brom; insbesondere aber Fluor und Chlor.

$C_1$-$C_4$-Alkyl umfasst Methyl, Ethyl, n-Propyl, iso-Propyl, n-Butyl, sek-Butyl, iso-Butyl und tert-Butyl.

Die erfindungsgemässen $C_3$-$C_5$-Alkenyloxy bzw. $C_3$-$C_5$-Alkinyloxyreste sind vorzugsweise über ein gesättigtes Kohlenstoffatom an das Sauerstoffatom gebunden. Zu nennen sind insbesondere der Allyloxy- und der Propargyloxy` rest.

Mit Halogenalkyl sind die ganz oder teilweise, gleich oder unterschiedlich halogensubstituierten Alkylradikale gemäss der jeweiligen Definitionsbreite gemeint. Als Halogensubstituenten sind Fluor und Chlor bevorzugt. Zu nennen sind Reste, wie etwa 2,2,2-Trifluorethyl, 1,1,1,3,3,3-Hexafluoroprop-2-yl, Trifluormethyl, Difluormethyl, Trichlormethyl, Dichlormethyl oder Difluormethyl.

In den aus mehreren Grundelementen zusammengesetzten Substituenten, wie etwa $C_1$-$C_4$-Alkoxy, $C_1$-$C_4$-Halogenalkoxy, $C_1$-$C_4$-Alkylthio, $C_1$-$C_4$-Halogenalkylthio oder $C_1$-$C_4$-Alkoxy-$C_1$-$C_4$-alkoxy, können die jeweiligen Teilelemente innerhalb ihrer Definitionsbreite frei gewählt werden.

Anmeldungsgemäss bedeutet der Substituent $R^4$ ein mit 1 bis 5 Fluoratomen substituierter $C_1$-$C_2$-Alkylrest, er steht somit für einen Rest ausgewählt aus der Gruppe: $CF_3$, $CHF_2$, $CH_2F$, $CHFCH_3$, $CHFCH_2F$, $CHFCHF_2$, $CHFCF_3$, $CF_2CH_3$, $CF_2CH_2F$, $CF_2CHF_2$, $CF_2CF_3$, $CH_2CH_2F$, $CH_2CHF_2$ und $CH_2CF_3$,

Formel I umfasst die nachstehend angegebene Verbindung der Formel Ia:

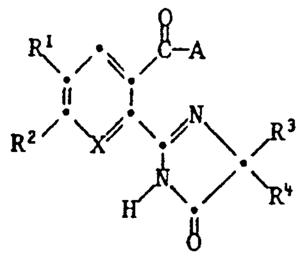

Ia

Des weiteren können die Reste $R^1$ und $R^2$ gemeinsam mit den Kohlenstoffatomen des Sechsringes, an welchen sie gebunden sind, einen anellierten ungesättigten oder teilweise ungesättigten carbocyclischen 5 oder 6-Ring bilden. In Abhängigkeit von der Bedeuteung von X ist das Formel I zugrunde liegende System dann ein Naphthalin-, Dihydronaphthalin- oder Tetrahydronaphthalin-system oder ein Indan- oder Dihydroindansystem, während bei der Bedeutung X = N der Formel I zugrunde liegende Heterocyclus ein Chinolin-, Dihydrochinolin-, Tetrahydrochinolin-, Cyclopenta[b]pyridin-,oder Dihydrocyclopenta[b]pyridin-System bildet.

Das Substituentenpaar $R^1/R^2$ kann aber auch für einen anellierten heterocyclischen 5 oder 6 Ring mit bis zu 2 Sauerstoff oder Schwefelatomen stehen.

Wenn X für N steht bilden diese anellierten Teilringe zusammen mit dem Pyridinring beispielsweise ein Thiopheno[2,3-b]pyridin-, Thiopheno-[3,2-b]pyridin-, Dihydro-thiopheno[2,3-b]pyridin-, Dihydro-thiopheno-[3,2-b]pyridin,Dioxolo[4,5-b]pyridin-, Dihydro-pyrido[2,3b]1,4-dioxin-, Pyrido[2,3-d]1,3-dioxin, Furo[2,3-b]-pyridin-, Furo[3,2-b]-pyridin-, Pyrano[3,4-b]pyridin-, Dihydropyrano[3,4-b]pyridin, Pyrano-[4,3-b]pyridin oder Dihydropyrano[4,3-b]pyridin-system. Wenn X für CH steht, bildet das anellierte Substituentenpaar $R^1/R^2$ zusammen mit dem Sechsring beispielsweise ein Benzo[b]furan-, Benzo[b]1,4-dioxin-, Benzo[b]thiophen-, Dihydro-benzo[b]furan-, Dihydro-benzo[b]thiophen-, Dihydrobenzo[b]1,4-dioxin-, Dihydro-benzo[c]thiophen, Dihydro-benzo[c]furan-, Chroman-, Isochroman-, Chromen- oder Isochromensystem.

Als Kation $M^{\oplus}$ kommen sowohl die Alkali- bzw. Erdalkaliionen von Li, Na, K, Rb, Mg oder Ca in Frage, wie auch quarternäre oder protonierte stickstoffhaltige Basen wie $NH_4^{\oplus}$, $[N(H)_m(R)_n]^{\oplus}$ (mit $m+n=4$ und R = $C_1$-$C_6$-Alkyl, gegebenenfalls substituiert durch Hydroxy, oder $C_1$-$C_6$-Alkoxy), oder heterocyclische Basen wie Morpholin oder Piperidin. Zu nennen sind unter anderem $NH(C_2H_5)_3^{\oplus}$ oder $NH(CH_2CH_2OH)_3^{\oplus}$.

Die Verbindungen der Formel I können in unterschiedlichen isomeren und tautomeren Formen vorliegen. Diese Isomeren bzw. Tautomeren, wie auch die Kombinationen unterschiedlicher Stereoisomere werden ebenfalls von Formel I umfasst.

Insbesondere betrifft die Erfindung Verbindungen der Formel I, worin

| | |
|---|---|
| X | CH oder N |
| $R^1$ und $R^2$ | unabhängig voneinander Wasserstoff oder $C_1$-$C_4$-Alkyl; oder |
| $R^1$ und $R_2$ | gemeinsam mit den Kohlenstoffatomen des Sechsringes an welchen sie gebunden sind einen ungesättigten oder teilweise ungesättigten carbocyclischen oder einen ein O oder S Atom enthaltenden heterocyclischen 5- oder 6-Ring bilden; und |
| A | OH; $O^{\ominus}M^{\oplus}$; $C_1$-$C_4$-Alkoxy; $C_3$-$C_5$-Alkenyloxy; $C_3$-$C_5$-Alkinyloxy; und |
| $M^{\oplus}$ | ein Kationenäquivalent eines Alkali- oder Erdalkalimetalls; oder einer stickstoffhaltigen Base; und |
| B | ein Imidazolon der Formel |

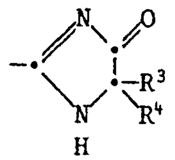

bilden; und

$R^3$ $C_1$-$C_4$-Alkyl; und

$R^4$ ein mit 1 bis 5 Fluoratomen substituierten Methyl- oder Ethylrest bedeutet.

Bevorzugt sind Verbindungen der Formel I, worin

X CH oder N;

$R^1$ und $R^2$ unabhängig voneinander Wasserstoff oder $C_1$-$C_4$-Alkyl; oder

$R^1$ und $R^2$ gemeinsam mit den Kohlenstoffatomen des Sechsringes an welchen sie gebunden sind einen Cyclopenten-, Cyclohexen-, Benzol-oder Dihydropyranrest bilden; und

A OH; $C_1$-$C_4$-Alkoxy;

B ein Imidazolon der Formel

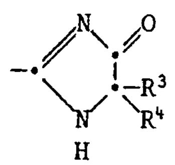

bilden; und

$R^3$ $C_1$-$C_4$-Alkyl; und

$R^4$ ein mit 1 bis 5 Fluoratomen substituierten Methyl- oder Ethylrest bedeutet.

Besonders bevorzugt sind die Verbindungen der Formel I, in denen X, $R^1$, $R^2$, A, B und $R^3$ wie zuvor definiert sind und in denen $R^4$ für $CHF$-$CH_3$, $CF_3$ oder $CHF_2$ steht.

Hervorzuheben sind auch die Verbindungen der Formel I, in denen X, $R^1$, $R^2$, A, B und $R^4$ wie zuvor definiert sind und in denen $R^3$ für Methyl steht.

Hervorzuheben sind ausserdem die Verbindungen der Formel I, in denen $R^2$ Wasserstoff und $R^1$ $C_1$-$C_4$-Alkyl, insbesondere Methyl und Ethyl, bevorzugt Ethyl, bedeutet.

Ebenfalls hervorzuheben sind die Verbindungen der Formel I, in denen $R^1$ und $R^2$ gemeinsam für eine $-(CH_2)_3-$, $-(CH_2)_4-$ oder $CH=CH-CH=CH$-Brücke stehen.

Namentlich zu nennen sind:

2-(5-Methyl-5-trifluormethyl-imidazol-4-on-2-yl)-isonicotinsäure,
2-(5-Methyl-5-trifluormethyl-imidazol-4-on-2-yl)-5-methyl-isonicotinsäure,
2-(5-Methyl-5-trifluormethyl-imidazol-4-on-2-yl)-5-ethyl-isonicotinsäure,
2-[5-(1-Fluorethyl)-5-methyl-imidazol-4-on-2-yl]-isonicotinsäure,
2-[5-(1-Fluorethyl)-5-methyl-imidazol-4-on-2-yl]-5-methyl-isonicotinsäure,
2-(5-Methyl-5-trifluormethyl-imidazol-4-on-2-yl)-chinolin-3-carbonsäure oder
2-[5-(1-Fluorethyl)-5-methyl-imidazol-4-on-2-yl]-5-ethyl-isonicotinsäure,
2-[5-Difluormethyl-5-methyl-imidazol-4-on-2-yl]-isonicotinsäure,
2-[5-Difluormethyl-5-methyl-imidazol-4-on-2-yl]-5-methyl-isonicotinsäure,
2-[5-Difluormethyl-5-methyl-imidazol-4-on-2-yl]-5-ethyl-isonicotinsäure,
2-[5-Difluormethyl-5-methyl-imidazol-4-on-2-yl]-5,6-dihydro-cyclopenta[b]pyridin-3-carbonsäure,
2-[5-Difluormethyl-5-methyl-imidazol-4-on-2-yl]-5,6,7,8-tetrahydrochinolin-3-carbonsäure,
2-[5-Difluormethyl-5-methyl-imidazol-4-on-2-yl]-chinolin-3-carbonsäure,
2-[5-(1-Fluorethyl)-5-methyl-imidazol-4-on-2-yl]-5,6-dihydrocyclopenta[b]pyridin-3-carbonsäure,
2[5-(1-Fluorethyl)-5-methyl-imidazol-4-on-2-yl]5,6,7,8-tetrahydrochinolin-3-carbonsäure,
2-[5-(1-Fluorethyl)-5-methyl-imidazol-4-on-2-yl]-isonicotinsäuremethylester,
2-[5-(1-Fluorethyl)-5-methyl-imidazol-4-on-2-yl]-isonicotinsäureethylester und
2-[5-(1-Fluorethyl)-5-methyl-imidazol-4-on-2-yl-]-5-ethyl-isonicotinsäure-ethylester,

Aufgrund ihrer chemischen Struktur können die anmeldungsgemässen Verbindungen der Formel I definiert

werden als:
Imidazolone der Formel Ia

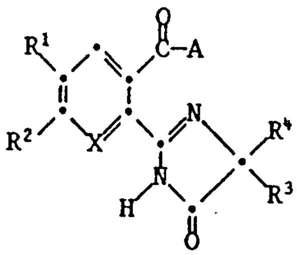

Ia,

worin $R^1$, $R^2$, A, $R^3$ und $R^4$ wie zuvor definiert sind.

Die anmeldungsgemässen Verbindungen der Formel I können analog zu literaturbekannten Verfahren hergestellt werden.

Die Erfindung betrifft somit auch Verfahren zur Herstellung von Verbindungen der Formel Ia

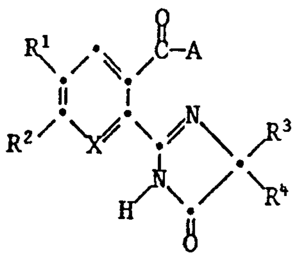

Ia,

worin A für OH steht und die Reste X, $R^1$, $R^2$, $R^3$ und $R^4$ wie zuvor definiert sind, dadurch gekennzeichnet, dass man

a) einen Diester der Formel IV

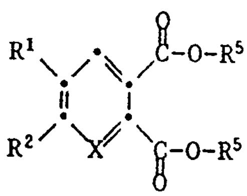

IV,

worin X, $R^1$ und $R^2$ wie zuvor definiert sind und die Reste $R^5$ unabhängig voneinander für $C_1$-$C_6$-Alkyl stehen mit einem α-Aminosäureamid der Formel II in der $R^3$ und $R^4$ wie zuvor definiert sind

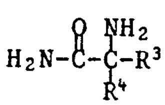

II

in Gegenwart einer mindestens äquimolaren Menge einer Base umsetzt und das Reaktionsgemisch anschliessend hydrolytisch aufarbeitet.

Die Erfindung umfasst auch Verfahren zur Herstellung von Salzen der Formel Ia'

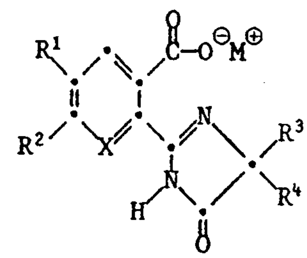

Ia',

worin X, $R^1$, $R^2$, $R^3$ und $R^4$ wie zuvor definiert sind und $M^{\oplus}$ für ein Kationenäquivalent eines Alkali- oder Erdalkalimetalls oder einer stickstoffhaltigen Base steht,
dadurch gekennzeichnet, dass man
f) eine Verbindung der Formel Ia

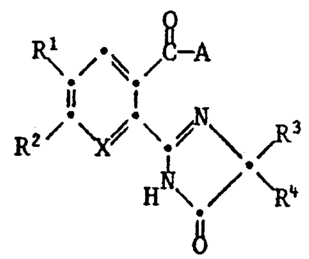

Ia,

in der die Reste X, $R^1$, $R^2$, und $R^4$ wie zuvor definiert sind und A für OH steht mit einer äquivalenten Menge einer Verbindung der Formel VII

$OH^{\ominus}M^{\oplus}$ VII,

in der $M^{\oplus}$ für ein Kationenäquivalent eines Alkali- oder Erdalkalimetalls oder eine quaternäre stickstoffhaltige Base steht, umsetzt, oder
g) eine Verbindung der Formel Ia, in der A für OH steht mit einem Alkali- oder Erdalkalimetall oder einem Alkali oder Erdalkalisalz oder der stickstoffhaltigen Base direkt umsetzt.

Zu den erfindungsgemässen Verfahren analoge Umsetzungen, sowie die bei deren Durchführung zu beachtenden Reaktionsbedingungen, wie Auswahl von Lösungsmitteln und Reaktanden, sind beispielsweise aus den folgenden Patentpublikationen bekannt geworden: EP-A-0041623, EP-A-0212200, GB-A-2174395, DE-A-3420271, EP-A-0133309, EP-A-0216360.

Die Umsetzungen erfolgen im allgemeinen zwischen 0 und 180 ° C, vorzugsweise im Temperaturbereich zwischen Raumtemperatur und der Rückflusstemperatur der Reaktionsgemische.

Bevorzugt werden die Reaktionen in Gegenwart von Lösungsmitteln durchgeführt. Geeignete Lösungsmittel sind insbesondere diejenigen, welche die Edukte und/oder Produkte zu lösen vermögen und welche die Reaktion nicht nachteilig beeinflussen.

Zu nennen sind Alkohole, wie Methanol, Ethanol, n-Propanol, Iso-Propanol, n-Butanol, tert.-Butanol, Glyme oder Ethylenglycol; Ether, wie Diethylether, Tetrahydrofuran oder Diglyme; Halogenkohlenwasserstoffe, wie Dichlormethan, Trichlormethan oder Tetrachlormethan; aromatische Kohlenwasserstoffe, wie Benzol, Toluol, Xylol, Chlorbenzol oder Dichlorbenzol; Säureamide, wie Dimethylformamid, Acetamid, N-Methylpyrrolidon; Sulfoxide, wie Dimethylsulfoxid oder Sulfolan; oder andere gegenüber den Reaktionspartnern inerte Lösungsmittel.

In einigen der zuvor genannten Reaktionen ist es vorteilhaft wasserfrei zu arbeiten während in Verfahren f Wasser allein als Lösungsmittel oder Wasser-Lösungsmittelgemische zur Anwendung kommen können.

Geeignete Basen sind u.a. Alkoholate, Amine, Hydride, Amide oder metallorganische Verbindungen.

Protonensäuren sind Halogenwasserstoffsäuren, Schwefelsäure oder Phosphorsäure.

Die Ausgangsverbindungen der Formel IV sind bekannt oder können analog zu literaturbekannten Verfahren hergestellt werden. Es wird insbesondere auf nachstehene Patentpublikationen, sowie die dort zitierte Literatur verwiesen: EP-A-0041623, EP-A-0212200, GB-A-2174395, DE-A-3420271, EP-A-0133309, EP-A-0216360, EP-A-0172140 sowie auf die noch unveröffentlichte Schweizer Patentanmeldung CH 2301/87-1 vom 18.6.1987.

Die α-Aminosäureamide der Formel II sind neu. Sie sind als Zwischenprodukte insbesondere für die Synthese der erfindungsgemässen Verbindungen der Formel I geeignet, zu welchen sie einen wesentlichen Strukturbeitrag leisten.

Die Erfindung betrifft somit auch die neuen α-Aminosäureamide der Formel II

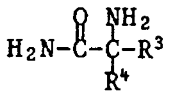

II,

worin

$R^3$ $C_1$-$C_4$-Alkyl und

$R^4$ einen mit 1 bis 5-Fluoratomen substituierten Methyl- oder Ethylrest bedeutet.

Die α-Aminosäureamide der Formel II können in unterschiedlichen stereoisomeren Formen vorliegen. Die Erfindung betrifft sowohl die racemischen, wie auch die verschiedenen stereoisomeren Formen.

Die α-Aminosäureamide der Formel II können analog zu literaturbekannten Verfahren hergestellt werden.

Insbesondere durch Amidierung von Aminosäuren der Formel VIII

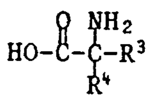

VIII,

worin $R^3$ und $R^4$ wie zuvor definiert sind z.B. gemäss nachstehender Reaktionfolge durch Umsetzung der Aminosäure VIII mit Trifluoressig-

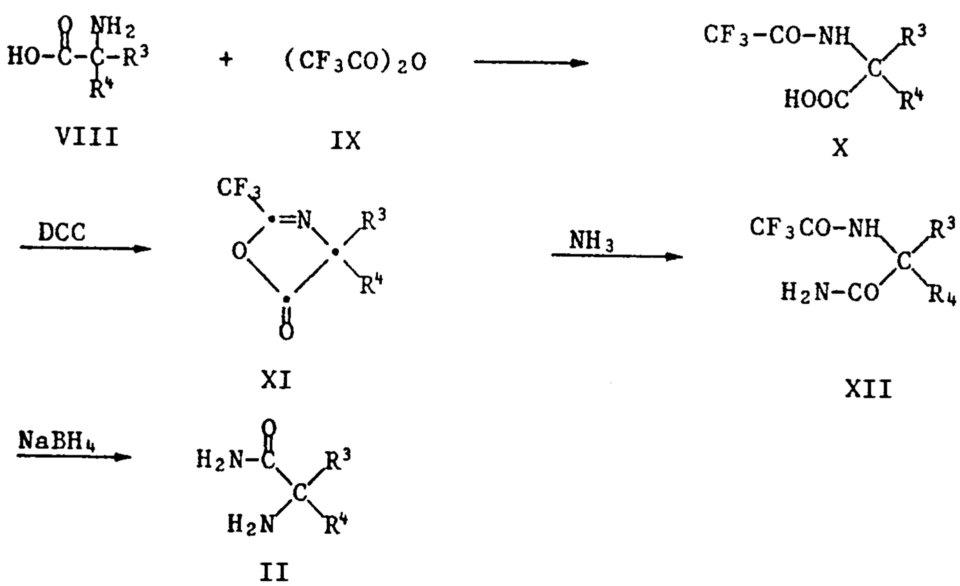


VIII IX X XI XII II

säureanhydrid IX zu der N-geschützen Verbindung X und nachfolgender Cyclisierung mit Dicyclohexylcarbodiimid (DCC) zum Oxazolidinon XI welches mit $NH_3$ unter Ringöffnung zum Amid XII reagiert. Aus XII kann dann die Trifluoracetylschutzgruppe z.B. mit $NaBH_4$ abgespalten werden (analog zu Jones et al J. Chem. Soc. 1965 6227-39 und E. Wünsch in Houben Weyl Bd. 15/1 S. 177 ff.)

Ein weiterer genereller Zugang zu den α-Aminosäureamiden II eröffnet die Strecker-Synthese gemäss nachfolgenden Reaktionsschema,

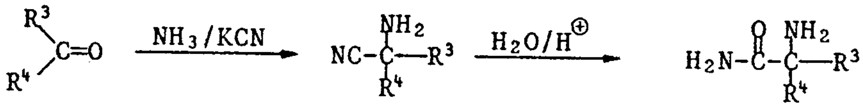


XIII XIV II

in der II ausgehend von dem Keton der Formel XIII, in dem $R^3$ und $R^4$ wie zuvor definiert sind, durch Addition von Ammoniak und Cyanid das Nitril XIV erhältlich ist, aus welchem dann durch Hydrolyse das α-Aminosäureamid II dargestellt werden kann (z.B. nach Biochim. Biophys. Acta 74 (1963) 386-391).

Die zu diesem Verfahren benötigten fluorierten Ketone der Formel XIII sind bekannt oder können analog zu literaturbekannten Verfahren synthetisiert werden (etwa durch Grignard Reaktionen analog zu Olah; Chem. Ber. 89 [1956] 864 oder Haszeldine; J. Chem Soc. 1954 1273).

Die Aminosäuren der Formel VIII sind ebenfalls bekannt oder können analog zu literaturbekannten Verfahren hergestellt werden. Die neuen Aminosäuren VIII, die neuen Ketone XIII und die Verbindungen der Formel XIV sind ebenfalls Gegenstand der vorliegenden Erfindung.

So durch Fluorierung entsprechend substituierter ß-Hydroxyaminosäuren XIX mit $HF/SF_4$ bei tiefen Temperaturen (analog zu Kollonitsch; J. Org. Chem. 44 [1979] 771-7 und J. Org. Chem. 40 [1975] 3808-9). Dieses Verfahren ist insbesondere geeignet zur Herstellung von Verbindungen der Formel VIIIa, in denen $R^4$ für einen Rest der Formel $R^6$ CHF (mit $R^6 = CH_3$, $CH_2F$, $CHF_2$ und $CF_3$) steht. Dieses Verfahren geht aus von

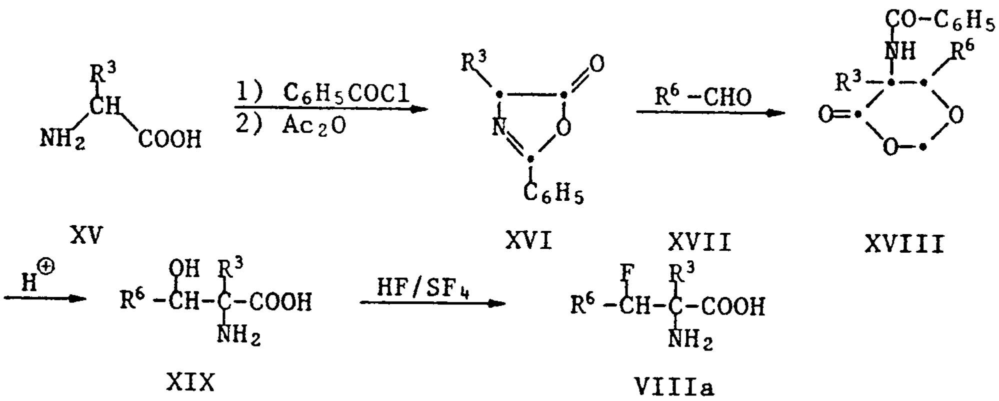


XV XVI XVII XVIII XIX VIIIa

Aminosäuren der Formel XV, die mit Benzoylchlorid und Acetanhydrid zum Oxazolon XVI cyclisiert der dann durch Umsetzung mit einem Aldehyd XVII zu XVIII umlagert. Unter Säureeinwirkung kann danach die ß-Hydroxyaminosäure XIX erhalten werden (analog zu Kaminski; Synthesis 1973 792-4).

Die α-Aminosäuren VIIIa können aber auch durch eine Variante der Erlenmayer'schen Azlactonsynthese (anolog zu F. Heinzer und D. Bellus, Helv. Chim. Acta 64 (1981) 2279) durch Umsetzung eines Isocyanoessigsäureesters XXIII (mit $R^7 = C_1$-$C_4$-Alkyl) mit einem Aldehyd ($R^6$ CHO) zu dem Oxazolin XXIV hergestellt werden, wobei das Oxazolin XXIV mit Wasser unter Ringöffnung zu der ß-Hydroxyaminosäure XIX reagiert. Die Aminosäure XIX kann dann, wie oben gezeigt, fluoriert werden.

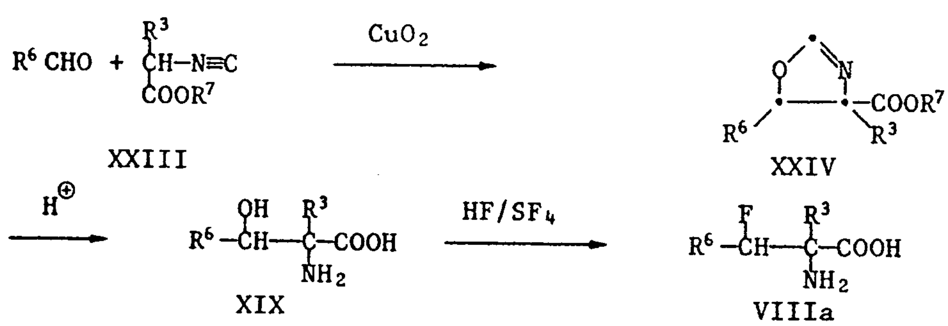


XXIII XXIV XIX VIIIa

Des weiteren können die Aminosäuren der Formel VIII hergestellt werden durch die Umsetzung der

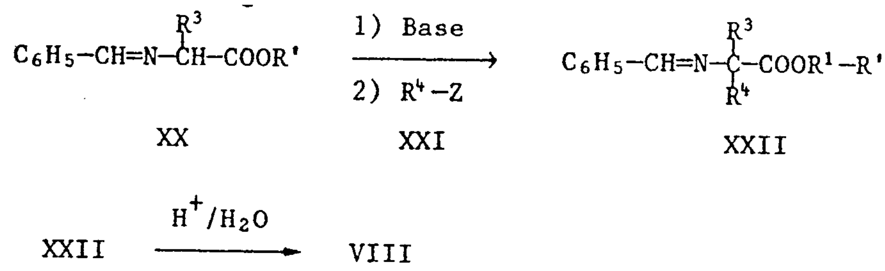


XX XXI XXII

XXII VIII

Schiff'schen Base XX, in der R' für $C_1$-$C_4$-Alkyl steht. Die Verbindung der Formel XX reagiert unter Baseneinwirkung mit der Verbindung XXI, in der Z für eine nucleofage Gruppe, wie Chlor oder Brom steht, zum Ester XXII, aus welchem durch saure Hydrolyse VIII erhältlich ist (analog zu Bey et al.; J. Org. Chem. 44 [1979], 2732).

Ein weiterer Gegenstand der Erfindung sind herbizide Mittel enthaltend mindestens eine Verbindung der Formel I als Aktivsubstanz, die Verwendung von Verbindungen der Formel I in Verfahren zur pre-und/oder postemergenten Bekämpfung von Unkräutern und Gräsern sowie die Herstellung herbizider Mittel enthaltend Verbindungen der Formel I.

Die Wirkstoffe der Formel I werden in der Regel mit Aufwandmengen von 0,005 bis 5, insbesondere 0,05 bis 4,0 kg/ha, insbesondere 0,1 bis 2,0 kg/ha, erfolgreich eingesetzt. Die für den jeweiligen Verwendungszweck und Anwendungszeitpunkt optimale Wirkstoffmenge bei preemergenter bzw. postemergenter Anwendung kann durch Versuche ermittelt werden. Des weiteren können die Verbindungen der Formel I auch auf das Saatgut der jeweiligen Kulturpflanze aufgebracht werden (Saatgutbeizung).

Die Verbindungen der Formel I werden vorgeschlagen zum Einsatz in Nutzpflanzenkulturen, insbesondere in Getreide, Baumwolle, Soja, Mais, Reis und Sorghum. Die Verbindungen der Formel I zeichnen sich durch Selektivität bei Getreide, Leguminosen und Soja aus.

Die herbizid wirksamen Aktivsubstanzen der Formel I können auch auf das Saatgut der Nutzpflanzenkultur aufgetragen werden (Samenbeizung). Die Aktivsubstanz wird dann mit der Aussaat der Nutzpflanze auf das Feld gebracht. Das mit der herbizid wirksamen Aktivsubstanz der Formel I behandelte Saatgut ist ein weiterer Gegenstand der Erfindung.

Die Verbindungen der Formel I werden in unveränderter Form oder vorzugsweise als Mittel zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dicotyl-phthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder -ethylether, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der

Formel I nichtionogene, kation- und/oder anionaktive Tenside mit guten Emulgier-, Dispergier-und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können, genannt. Ferner sind auch die Fettsäure-methyl-laurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali-oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2-Sulfonsäuregruppen und einen Fettsäurerest mit 8-22 C-Atomen.

Alkylarylsulfonate sind z.B. die Na-, Ca- oder Triethanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Ethylenoxid-Adduktes oder Phospholipide in Frage.

Als nicht ionische Tenside kommen in erster Linie Polyglykolether und -esterderivate von aliphatischen oder cycloaliphatischen Alkoholen, gesättigten oder ungesättigten Fettsäuren und Alkylphenolen in Frage, die 3 bis 10 Glykolethergruppen und 8 bis 20 Kohlensotffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Ethylenglykolethergruppen und 10 bis 100 Propylenglykolethergruppen enthaltenden Polyethylenoxidaddukte an Polypropylenglykol, Ethylendiaminopolypropylenglykol und Alkylpolypropylenglykol mit 1 bis 10 Kohlenstoffdatomen in der Alkylkette. Die genannten Verbindungen enthalten üblicherweise pro Propylenglykol-Einheit 1 bis 5 Ethylenglykoleinheiten.

Als Beispiele nichtionischer Tenside seien Nonylphenolpolyethoxyethanole, Ricinusölpolyglykolether, Polypropylen-Polyethylenoxidaddukte, Tributylphenoxypolyethanol Polyethylenglykol und Octylphenoxypolyethoxyethanol erwähnt.

Ferner kommen auch Fettsäureester von Polyoxyethylensorbitan wie das Polyoxyethylensorbitan-trioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Ethylsulfate vor, z.B. das Stearyltrimethylammoniumchlorid oder das Benzyldi(2-chlorethyl)-ethylammoniumbromid.

Die in der Formulierungstechnik gebräuchlichen Tenside sind u.a. in folgenden Publikationen beschrieben:

"1986 International Mc Cutcheon's Emulsifiers & Detergents" Glen Rock, NJ, USA, 1986.
Dr, Helmut Stache "Tensid Taschenbuch"
Carl Hanser Verlag, München/Wien 1981.

Die herbiziden Zubereitungen enthalten in der Regel 0,1 bis 95 %, insbesondere 0,1 bis 80 %, Wirkstoff der Formel I, 1 bis 99,9 % eines festen oder flüssigen Zusatzstoffes und 0 bis 25 %, insbesondere 0,1 bis 25 % eines Tensides.

Insbesondere setzen sich bevorzugte Formulierungen folgendermassen zusammen: (% = Gewichtsprozent)

| Emulgierbare Konzentrate: | |
|---|---|
| Wirkstoff der Formel I: | 1 bis 20 % bevorzugt 5 bis 10 % |
| oberflächenaktives Mittel: | 5 bis 30 %,vorzugsweise 10 bis 20 % |
| flüssiges Trägermittel: | 50 bis 94 %,vorzugsweise 70 bis 85 %. |

| Stäube: | |
|---|---|
| Wirkstoff der Formel I:<br>festes Trägermittel: | 0,1 bis 10 %, vorzugsweise 0,1 bis 1 %<br>99,9 bis 90 %, vorzugsweise 99,9 bis 99 %. |

| Suspensions-Konzentrate: | |
|---|---|
| Wirkstoff der Formel I:<br>Wasser:<br>oberflächenaktives Mittel: | 5 bis 75 %, vorzugsweise 10 bis 50 %<br>94 bis 25 %, vorzugsweise 90 bis 30 %<br>1 bis 40 %, vorzugsweise 2 bis 30 %. |

| Benetzbare Pulver: | |
|---|---|
| Wirkstoff der Formel I:<br>oberflächenaktives Mittel:<br>festes Trägermittel: | 0,5 bis 90 %, vorzugsweise 1 bis 80 %<br>0,5 bis 20 %, vorzugsweise 1 bis 15 %<br>5 bis 95 %, vorzugsweise 15 bis 90 %. |

| Granulate: | |
|---|---|
| Wirkstoff der Formel I:<br>festes Trägermittel: | 0,5 bis 30 %, vorzugsweise 3 bis 15 %<br>99,5 bis 70 %, vorzugsweise 97 bis 85 %. |

Während als Handelsware eher konzentrierte Mittel bevorzugt werden, verwendet der Endverbraucher in der Regel verdünnte Mittel. Die Anwendungsformen können bis hinab zu 0,001 % an Wirkstoff verdünnt werden. Die Aufwandmengen betragen in der Regel 0,005 bis 5 kg As/ha.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Die nachstehenden Beispiele erläutern den Erfindungsgegenstand.

Beispiel 1: 2-Amino-3-fluor-2-methylbuttersäure

In einem durch Trockeneis/Aceton auf -56 °C gekühlten Monelkessel werden 40 g (0,3 Mol) 2-Amino-3-hydroxy-2-methylbuttersäure vorgelegt. Danach werden 400 g (20 Mol) Fluorwasserstoff einkondensiert. Nach Abklingen der exothermen Reaktion wird portionsweise unter Rühren mit 106 g (1 Mol) Schwefeltetrafluorid versetzt. Das Reaktionsgemisch wird dann noch 22 Stunden bei -66 °C und einem Druck von $2 \cdot 10^5$ Pascal weitergerührt und danach am Rotationsverdampfer eingeengt. Der so erhältliche Rückstand wird an einem Ionenaustauscher (Dowex 50 W x 8 150-200 mesh) mit Wasser und 1 molarer Salzsäure als Eluiermittel gereinigt. Die so erhältliche wässrige Lösung wird zur Trockene eingeengt, in 150 ml Ethanol aufgenommen und durch Zusatz von 100 ml Propylenchlorid gefällt. Das Produkt wird abfiltriert und im Vakuum bei 50 °C getrocknet.

Man isoliert 23,6 g (58,1 %) der Titelverbindung der Formel

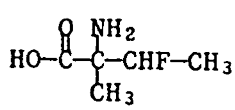

als weisses Pulver vom Smp. 226 °C.

Beispiel 2: 3-Fluor-2-methyl-2-trifluoracetamino-buttersäure

1,35 g (10 mMol) der nach Beispiel 1 erhältlichen 2-Amino-3-fluor-2-methylbuttersäure werden unter

Rühren bei Raumtemperatur portionsweise in 6,5 g (31 mMol) Trifluoressigsäureanhydrid eingetragen. Es wird noch weitere 2 Stunden bei Raumtemperatur nachgerührt und dann bei 25 °C am Rotationsverdampfer zur Trockene eingeengt. Der Rückstand wird in 20 ml Eiswasser, welches mit einem Tropfen Pyridin versetzt ist, aufgenommen und danach mit Diethylether extrahiert.

Nach dem Eindampfen der organischen Phase erhält man ein langsam kristallisierendes Oel.

Man isoliert 1,8 g (78,3 %) der Titelverbindung der Formel

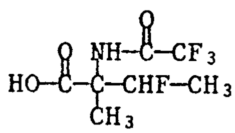

als Kristalle vom Smp. 25-30 °C.

| Elementaranalyse [%]: | | | | |
|---|---|---|---|---|
| Ber.:<br>Gef.: | C : 36,37<br>C : 36,42 | H : 3,93<br>H : 3,94 | N : 6,06<br>N : 6,03 | F : 32,88<br>F : 32,50 |

Beispiel 3a: 2-Amino-3-fluor-2-methylbuttersäureamid

Zu einer Lösung von 23,1 g (0,1 Mol) 3-Fluor-2-methyl-2-trifluoracetamino-buttersäure (erhältlich nach Beispiel 2) in 200 ml Dichlormethan wird unter Rühren bei 25-30 °C eine Lösung von 20,6 g (0,1 Mol) N,N'-Di-cyclohexylcarbodiimid in 50 ml Dichlormethan getropft. Nach 2 Stunden wird bei 25 °C so lange Ammoniak-Gas eingeleitet, bis die Suspension über lange Zeit alkalisch bleibt. Nach Abfiltrieren und Eindampfen des Lösungsmittel erhält man 23 g eines braunen Oels, welches in 200 ml Ethanol gelöst wird. Dazu werden bei 25-30 °C unter Rühren portionsweise 15,1 g 0,4 M-Natriumborhydrid gegeben. Nach weiteren 5 Stunden wird unter Kühlen 100 ml Aceton zugetropft und nach einer weiteren Stunde am Rotationsverdampfer zur Trockene eingeengt. Der Rückstand wird mit 100 ml Wasser versetzt und mit Dichlormethan extrahiert. Aus der getrockneten organischen Phase erhält man nach dem Einengen das Produkt.

Man isoliert 11,7 g (87,3 %) der Titelverbindung der Formel

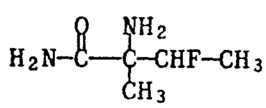

als gelbes Oel.

Beispiel 3b: Abtrennung eines Diasteromeren des 2-Amino-3-fluoro-2-methylbuttersäureamids

11,7 g des nach Beispiel 3a als gelbes Oel erhältlichen Diastereomerengemischs wird durch Umkristallisieren aus Essigester/Petrolether (95:5) aufgetrennt.

Man isoliert 1,2 g (8,9 %) eines reinen Diastereomeren der Titelverbindung als Kristalle vom Smp. 81 °C.

Durch Einengen der Mutterlauge gewinnt man 7,1 g (53 %) des Diastereomerengemischs als hellgelbes Wachs zurück.

Beispiel 3c: 2-Amino-3,3-difluor-2-methyl-propionsäureamid

Ausgehend von 2-Amino-3,3-difluor-2-methyl-propionsäure (Bey et al.; J. Org. Chem., 44 [1979], 2732) erhält man analog zu Beispiel 3a die Titelverbindung der Formel

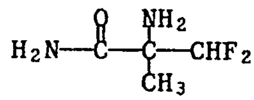

als Kristalle vom Smp. 77-80 ° C.

Beispiel 4: 2-Amino-2-trifluormethylpropionsäureamid

In einem Bombenrohr werden zu einer Suspension von 13 g (0,2 Mol) Kaliumcyanid in 10 ml Wasser unter Eiskühlung 19,2 ml (0,2 Mol) 1,1,1-Trifluoraceton getropft. Bei verschlossenem Rohr wird dann 15 Min. bei 70 ° C gerührt. Danach wird unter Eiskühlung 25 ml 25%ige Ammoniaklösung zugetropft und bei verschlossenem Rohr für weitere 5 Stunden bei 100 ° C gerührt.

Nach Eindampfen des Reaktionsgemischs erhält man ein dunkelbraunes Oel aus welchen das Produkt durch Sublimation bei 140 ° C und 13 Pascal und anschliessendes Umkristallisieren am Essigester erhalten wird.

Man isoliert 1,7 g (6,0 %) der Titelverbindung der Formel

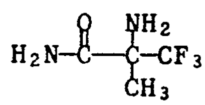

als weisses Pulver vom Smp. 84-85 ° C.

Beispiel 5: 2-(5-Methyl-5-trifluormethyl-1H-imidazol-4-on-2-yl)-pyridin-3-carbonsäure

Zu einer Lösung von 1,0 g (6,4 mMol) 2-Amino-2-trifluormethyl-propionsäureamid (erhältlich gemäss Beispiel 4) und 1,43 g (6,4 mMol) Pyridin-2,3-dicarbonsäurediethylester in 20 ml Toluol werden bei 60-80 ° C portionsweise 1,5 g (13,4 mMol) Kalium-tert-butylat gegeben. Nach 2 Stunden wird von dem Kaliumsalz abfiltriert, mit Ether gewaschen und in 15 ml Wasser aufgenommen. Die Lösung wird mit 2 molarer Salzsäure auf pH 4,5 gestellt und mit Essigester extrahiert. Die organische Phase wird getrocknet, eingeengt und der Rückstand aus Essigester umkristallisiert.

Man isoliert 0,55 g (30 %) der Titelverbindung der Formel

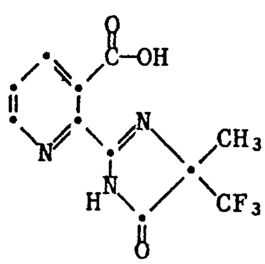

Verb. No. 1.001

als hellbeiges Pulver vom Smp. 180-184 ° C.

Gemäss Beispiel 5 können die nachfolgend in Tabelle 1 angegebenen Carbonsauren der Formel Ia hergestellt werden.

Tabelle 1

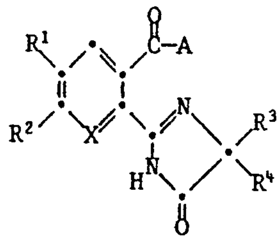

Ia

| Verb. Nr. | A | X | $R^1$ | $R^2$ | $R^3$ | $R^4$ | Smp. [°C] phys. Daten |
|---|---|---|---|---|---|---|---|
| 1.001 | OH | N | H | H | $CH_3$ | $CF_3$ | 180-184 |
| 1.002 | OH | N | $CH_3$ | H | $CH_3$ | $CF_3$ | 225-226 |
| 1.003 | OH | N | $C_2H_5$ | H | $CH_3$ | $CF_3$ | 223-225 |
| 1.004 | OH | N | n-$C_3H_7$ | H | $CH_3$ | $CF_3$ | |
| 1.005 | OH | N | iso-$C_3H_7$ | H | $CH_3$ | $CF_3$ | |
| 1.006 | OH | N | n-$C_4H_9$ | H | $CH_3$ | $CF_3$ | |
| 1.007 | OH | N | $-(CH_2)_3-$ | | $CH_3$ | $CF_3$ | |
| 1.008 | OH | N | $-(CH_2)_4-$ | | $CH_3$ | $CF_3$ | |
| 1.009 | OH | N | $-CH_2OCH_2CH_2-$ | | $CH_3$ | $CF_3$ | |
| 1.010 | OH | N | -CH=CH-CH=CH- | | $CH_3$ | $CF_3$ | 200-204 |
| 1.011 | OH | N | $C_2H_5$ | $CH_3$ | $CH_3$ | $CF_3$ | |
| 1.012 | OH | CH | H | H | $CH_3$ | $CF_3$ | |
| 1.013 | OH | CH | $CH_3$ | H | $CH_3$ | $CF_3$ | |
| 1.014 | OH | CH | H | $CH_3$ | $CH_3$ | $CF_3$ | |
| 1.015 | OH | N | H | H | $CH_3$ | $CHF_2$ | 188-190 |
| 1.016 | OH | N | $CH_3$ | H | $CH_3$ | $CHF_2$ | 236-238 |
| 1.017 | OH | N | $C_2H_5$ | H | $CH_3$ | $CHF_2$ | 197-199 |
| 1.018 | OH | N | n-$C_3H_7$ | H | $CH_3$ | $CHF_2$ | |
| 1.019 | OH | N | iso-$C_3H_7$ | H | $CH_3$ | $CHF_2$ | |
| 1.020 | OH | N | n-$C_4H_9$ | H | $CH_3$ | $CHF_2$ | |

| Verb. Nr. | A | X | $R^1$ | $R^2$ | $R^3$ | $R^4$ | Smp. [°C] phys. Daten |
|---|---|---|---|---|---|---|---|
| 1.021 | OH | N | $-(CH_2)_3-$ | | $CH_3$ | $CHF_2$ | 161-168 |
| 1.022 | OH | N | $-(CH_2)_4-$ | | $CH_3$ | $CHF_2$ | 215-218 |
| 1.023 | OH | N | $-CH_2OCH_2CH_2-$ | | $CH_3$ | $CHF_2$ | |
| 1.024 | OH | N | -CH=CH-CH=CH- | | $CH_3$ | $CHF_2$ | 236-238 |
| 1.025 | OH | N | $C_2H_5$ | $CH_3$ | $CH_3$ | $CHF_2$ | |
| 1.026 | OH | CH | H | H | $CH_3$ | $CHF_2$ | |
| 1.027 | OH | CH | $CH_3$ | H | $CH_3$ | $CHF_2$ | |
| 1.028 | OH | CH | H | $CH_3$ | $CH_3$ | $CHF_2$ | |
| 1.029 | OH | N | H | H | $CH_3$ | $CH_2F$ | |
| 1.030 | OH | N | $CH_3$ | H | $CH_3$ | $CH_2F$ | |
| 1.031 | OH | N | $C_2H_5$ | H | $CH_3$ | $CH_2F$ | |
| 1.032 | OH | N | $n-C_3H_7$ | H | $CH_3$ | $CH_2F$ | |
| 1.033 | OH | N | $iso-C_3H_7$ | H | $CH_3$ | $CH_2F$ | |
| 1.034 | OH | N | $n-C_4H_9$ | H | $CH_3$ | $CH_2F$ | |
| 1.035 | OH | N | $-(CH_2)_3-$ | | $CH_3$ | $CH_2F$ | |
| 1.036 | OH | N | $-(CH_2)_4-$ | | $CH_3$ | $CH_2F$ | |
| 1.037 | OH | N | $-CH_2OCH_2CH_2-$ | | $CH_3$ | $CH_2F$ | |
| 1.038 | OH | N | -CH=CH-CH=CH- | | $CH_3$ | $CH_2F$ | |
| 1.039 | OH | N | $C_2H_5$ | $CH_3$ | $CH_3$ | $CH_2F$ | |
| 1.040 | OH | CH | H | H | $CH_3$ | $CH_2F$ | |
| 1.041 | OH | CH | $CH_3$ | H | $CH_3$ | $CH_2F$ | |
| 1.042 | OH | CH | H | $CH_3$ | $CH_3$ | $CH_2F$ | |
| 1.043 | OH | N | H | H | $CH_3$ | $CHFCH_3$ | 126-130 |
| 1.044 | OH | N | $CH_3$ | H | $CH_3$ | $CHFCH_3$ | 206-207 |
| 1.045 | OH | N | $C_2H_5$ | H | $CH_3$ | $CHFCH_3$ | 167-169 |
| 1.046 | OH | N | $n-C_3H_7$ | H | $CH_3$ | $CHFCH_3$ | |
| 1.047 | OH | N | $iso-C_3H_7$ | H | $CH_3$ | $CHFCH_3$ | |
| 1.048 | OH | N | $n-C_4H_9$ | H | $CH_3$ | $CHFCH_3$ | |
| 1.049 | OH | N | $-(CH_2)_3-$ | | $CH_3$ | $CHFCH_3$ | 118-123 |
| 1.050 | OH | N | $-(CH_2)_4-$ | | $CH_3$ | $CHFCH_3$ | 117-132 |

| Verb. Nr. | A | X | $R^1$ | $R^2$ | $R^3$ | $R^4$ | Smp. [°C] phys. Daten |
|---|---|---|---|---|---|---|---|
| 1.051 | OH | N | $-CH_2OCH_2CH_2-$ | | $CH_3$ | $CHFCH_3$ | |
| 1.052 | OH | N | $-CH=CH-CH=CH-$ | | $CH_3$ | $CHFCH_3$ | 228-231 |
| 1.053 | OH | N | $C_2H_5$ | $CH_3$ | $CH_3$ | $CHFCH_3$ | |
| 1.054 | OH | CH | H | H | $CH_3$ | $CHFCH_3$ | |
| 1.055 | OH | CH | $CH_3$ | H | $CH_3$ | $CHFCH_3$ | |
| 1.056 | OH | CH | H | $CH_3$ | $CH_3$ | $CHFCH_3$ | |
| 1.057 | OH | N | H | H | $C_2H_5$ | $CF_3$ | |
| 1.058 | OH | N | $CH_3$ | H | $C_2H_5$ | $CF_3$ | |
| 1.059 | OH | CH | H | H | $C_2H_5$ | $CF_3$ | |
| 1.060 | OH | N | $C_2H_5$ | H | $C_2H_5$ | $CHF_2$ | |
| 1.061 | OH | N | $n-C_3H_7$ | H | $C_2H_5$ | $CHF_2$ | |
| 1.062 | OH | N | $iso-C_3H_7$ | H | $C_2H_5$ | $CH_2F$ | |
| 1.063 | OH | N | $n-C_4H_9$ | H | $C_2H_5$ | $CH_2F$ | |
| 1.064 | OH | CH | $CH_3$ | H | $C_2H_5$ | $CH_2F$ | |
| 1.065 | OH | N | $-(CH_2)_3-$ | | $C_2H_5$ | $CHFCH_3$ | |
| 1.066 | OH | N | $-(CH_2)_4-$ | | $C_2H_5$ | $CHFCH_3$ | |
| 1.067 | OH | N | $-CH_2OCH_2CH_2-$ | | $C_2H_5$ | $CHFCH_3$ | |
| 1.068 | OH | N | $-CH=CH-CH=CH-$ | | $C_2H_5$ | $CHFCH_3$ | |
| 1.069 | OH | CH | H | $CH_3$ | $C_2H_5$ | $CHFCH_3$ | |
| 1.070 | OH | N | $C_2H_5$ | H | $n-C_3H_7$ | $CF_3$ | |
| 1.071 | OH | N | $n-C_3H_7$ | H | $n-C_3H_7$ | $CF_3$ | |
| 1.072 | OH | N | $iso-C_3H_7$ | H | $n-C_3H_7$ | $CHF_2$ | |
| 1.073 | OH | N | $n-C_4H_9$ | H | $n-C_3H_7$ | $CHF_2$ | |
| 1.074 | OH | N | $-(CH_2)_3-$ | | $n-C_3H_7$ | $CH_2F$ | |
| 1.075 | OH | N | $-(CH_2)_4-$ | | $n-C_3H_7$ | $CH_2F$ | |
| 1.076 | OH | N | H | H | $n-C_3H_7$ | $CHFCH_3$ | |
| 1.077 | OH | N | $CH_3$ | H | $n-C_3H_7$ | $CHFCH_3$ | |
| 1.078 | OH | N | $-CH_2OCH_2CH_2-$ | | $n-C_3H_7$ | $CHFCH_3$ | |
| 1.079 | OH | N | $-CH=CH-CH=CH-$ | | $n-C_3H_7$ | $CHFCH_3$ | |

| Verb. Nr. | A | X | $R^1$ | $R^2$ | $R^3$ | $R^4$ | Smp. [°C] phys. Daten |
|---|---|---|---|---|---|---|---|
| 1.080 | OH | N | iso-$C_3H_7$ | H | i-$C_3H_7$ | $CF_3$ | |
| 1.081 | OH | N | n-$C_4H_9$ | H | i-$C_3H_7$ | $CF_3$ | |
| 1.082 | OH | N | $-(CH_2)_3-$ | | i-$C_3H_7$ | $CHF_2$ | |
| 1.083 | OH | N | $-(CH_2)_4-$ | | i-$C_3H_7$ | $CHF_2$ | |
| 1.084 | OH | N | $-CH_2OCH_2CH_2-$ | | i-$C_3H_7$ | $CH_2F$ | |
| 1.085 | OH | N | -CH=CH-CH=CH- | | i-$C_3H_7$ | $CH_2F$ | |
| 1.086 | OH | N | H | H | i-$C_3H_7$ | $CHFCH_3$ | |
| 1.087 | OH | N | $CH_3$ | H | i-$C_3H_7$ | $CHFCH_3$ | |
| 1.088 | OH | N | $C_2H_5$ | H | i-$C_3H_7$ | $CHFCH_3$ | |
| 1.089 | OH | N | n-$C_3H_7$ | H | i-$C_3H_7$ | $CHFCH_3$ | |
| 1.090 | OH | N | $-(CH_2)_3-$ | | n-$C_4H_9$ | $CF_3$ | |
| 1.091 | OH | N | $-(CH_2)_4-$ | | n-$C_4H_9$ | $CF_3$ | |
| 1.092 | OH | N | $-CH_2OCH_2CH_2-$ | | n-$C_4H_9$ | $CHF_2$ | |
| 1.093 | OH | N | -CH=CH-CH=CH- | | n-$C_4H_9$ | $CHF_2$ | |
| 1.094 | OH | N | $C_2H_5$ | $CH_3$ | n-$C_4H_9$ | $CH_2F$ | |
| 1.095 | OH | CH | H | H | n-$C_4H_9$ | $CH_2F$ | |
| 1.096 | OH | N | H | H | n-$C_4H_9$ | $CHFCH_3$ | |
| 1.097 | OH | N | $CH_3$ | H | n-$C_4H_9$ | $CHFCH_3$ | |
| 1.098 | OH | N | $C_2H_5$ | H | $CH_3$ | $CHFCH_3$ | 174-175* |

*** Verbindung 1.098 ist analog Beispiel 5 unter Verwendung der Diastereomeren gemäss Beispiel 3b herstellbar**

Beispiel 6: Allgemeines Verfahren zur Herstellung von Estern der Formel Ia (A ≠ OH)

Eine Lösung einer Verbindung der Formel Ib in einer Base, wie Triethylamin oder Pyridin wird mit einer 1 bis 3 molaren Menge eines Alkohols der Formel ROH versetzt und für 2 bis 3 Tage bei Raumtemperatur belassen. Danach wird zur Trockene eingeengt und das so erhaltene Produkt gegebenenfalls durch Umkristallisieren oder chromatographische Methoden weiter gereinigt.

Gemäss Beispiel 6 können die nachfolgend in Tabelle 3 angegebenen Ester der Formel Ia hergestellt werden.

Tabelle 3

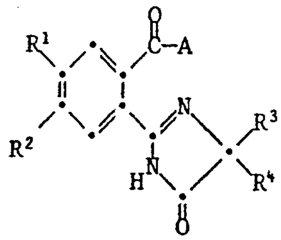

Ia

| Verb. Nr. | A | X | $R^1$ | $R^2$ | $R^3$ | $R^4$ | Smp. [°C] phys. Daten |
|---|---|---|---|---|---|---|---|
| 3.001 | $OCH_3$ | N | H | H | $CH_3$ | $CF_3$ | |
| 3.002 | $OC_2H_5$ | N | $CH_3$ | H | $CH_3$ | $CF_3$ | |
| 3.003 | $OCH_3$ | N | $C_2H_5$ | H | $CH_3$ | $CF_3$ | |
| 3.004 | $OC_2H_5$ | N | n-$C_3H_7$ | H | $CH_3$ | $CF_3$ | |
| 3.005 | $OCH_3$ | N | iso-$C_3H_7$ | H | $CH_3$ | $CF_3$ | |
| 3.006 | $OC_2H_5$ | N | n-$C_4H_9$ | H | $CH_3$ | $CF_3$ | |
| 3.007 | $OCH_3$ | N | $-(CH_2)_3-$ | | $CH_3$ | $CF_3$ | |
| 3.008 | $OC_2H_5$ | N | $-(CH_2)_4-$ | | $CH_3$ | $CF_3$ | |
| 3.009 | $OCH_3$ | N | $-CH_2OCH_2CH_2-$ | | $CH_3$ | $CF_3$ | |
| 3.010 | $OC_2H_5$ | N | -CH=CH-CH=CH- | | $CH_3$ | $CF_3$ | |
| 3.011 | $OC_2H_5$ | N | $C_2H_5$ | $CH_3$ | $CH_3$ | $CF_3$ | |
| 3.012 | $OCH_3$ | CH | H | H | $CH_3$ | $CF_3$ | |
| 3.013 | $OCH_3$ | CH | $CH_3$ | H | $CH_3$ | $CF_3$ | |
| 3.014 | $OCH_3$ | CH | H | $CH_3$ | $CH_3$ | $CF_3$ | |
| 3.015 | $OC_2H_5$ | N | H | H | $CH_3$ | $CHF_2$ | |
| 3.016 | $OCH_3$ | N | $CH_3$ | H | $CH_3$ | $CHF_2$ | |
| 3.017 | $OC_2H_5$ | N | $C_2H_5$ | H | $CH_3$ | $CHF_2$ | |
| 3.018 | $OC_2H_5$ | N | n-$C_3H_7$ | H | $CH_3$ | $CHF_2$ | |
| 3.019 | $OCH_3$ | N | iso-$C_3H_7$ | H | $CH_3$ | $CHF_2$ | |
| 3.020 | $OC_2H_5$ | N | n-$C_4H_9$ | H | $CH_3$ | $CHF_2$ | |

| Verb. Nr. | A | X | $R^1$ | $R^2$ | $R^3$ | $R^4$ | Smp. [°C] phys. Daten |
|---|---|---|---|---|---|---|---|
| 3.021 | $OC_2H_5$ | N | $-(CH_2)_3-$ | | $CH_3$ | $CHF_2$ | |
| 3.022 | $OCH_3$ | N | $-(CH_2)_4-$ | | $CH_3$ | $CHF_2$ | |
| 3.023 | $OC_2H_5$ | N | $-CH_2OCH_2CH_2-$ | | $CH_3$ | $CHF_2$ | |
| 3.024 | $OCH_3$ | N | $-CH{=}CH-CH{=}CH-$ | | $CH_3$ | $CHF_2$ | |
| 3.025 | $OC_2H_5$ | N | $C_2H_5$ | $CH_3$ | $CH_3$ | $CHF_2$ | |
| 3.026 | $OCH_3$ | CH | H | H | $CH_3$ | $CHF_2$ | |
| 3.027 | $OC_2H_5$ | CH | $CH_3$ | H | $CH_3$ | $CHF_2$ | |
| 3.028 | $OCH_3$ | CH | H | $CH_3$ | $CH_3$ | $CHF_2$ | |
| 3.029 | $OC_2H_5$ | N | H | H | $CH_3$ | $CH_2F$ | |
| 3.030 | $OCH_3$ | N | $CH_3$ | H | $CH_3$ | $CH_2F$ | |
| 3.031 | $OC_2H_5$ | N | $C_2H_5$ | H | $CH_3$ | $CH_2F$ | |
| 3.032 | $OCH_3$ | N | $n-C_3H_7$ | H | $CH_3$ | $CH_2F$ | |
| 3.033 | $OC_2H_5$ | N | $iso-C_3H_7$ | H | $CH_3$ | $CH_2F$ | |
| 3.034 | $OCH_3$ | N | $n-C_4H_9$ | H | $CH_3$ | $CH_2F$ | |
| 3.035 | $OC_2H_5$ | N | $-(CH_2)_3-$ | | $CH_3$ | $CH_2F$ | |
| 3.036 | $OCH_3$ | N | $-(CH_2)_4-$ | | $CH_3$ | $CH_2F$ | |
| 3.037 | $OC_2H_5$ | N | $-CH_2OCH_2CH_2-$ | | $CH_3$ | $CH_2F$ | |
| 3.038 | $OCH_3$ | N | $-CH{=}CH-CH{=}CH-$ | | $CH_3$ | $CH_2F$ | |
| 3.039 | $OC_2H_5$ | N | $C_2H_5$ | $CH_3$ | $CH_3$ | $CH_2F$ | |
| 3.040 | $OCH_3$ | CH | H | H | $CH_3$ | $CH_2F$ | |
| 3.041 | $OC_2H_5$ | CH | $CH_3$ | H | $CH_3$ | $CH_2F$ | |
| 3.042 | $OCH_3$ | CH | H | $CH_3$ | $CH_3$ | $CH_2F$ | |
| 3.043 | $OCH_3$ | N | H | H | $CH_3$ | $CHFCH_3$ | Harz |
| 3.044 | $OCH_3$ | N | $CH_3$ | H | $CH_3$ | $CHFCH_3$ | |
| 3.045 | $OCH_3$ | N | $C_2H_5$ | H | $CH_3$ | $CHFCH_3$ | |
| 3.046 | $OCH_3$ | N | $n-C_3H_7$ | H | $CH_3$ | $CHFCH_3$ | |
| 3.047 | $OC_2H_5$ | N | $iso-C_3H_7$ | H | $CH_3$ | $CHFCH_3$ | |
| 3.048 | $OCH_3$ | N | $n-C_4H_9$ | H | $CH_3$ | $CHFCH_3$ | |
| 3.049 | $OC_2H_5$ | N | $-(CH_2)_3-$ | | $CH_3$ | $CHFCH_3$ | |
| 3.050 | $OCH_3$ | N | $-(CH_2)_4-$ | | $CH_3$ | $CHFCH_3$ | |

| Verb. Nr. | A | X | $R^1$ | $R^2$ | $R^3$ | $R^4$ | Smp. [°C] phys. Daten |
|---|---|---|---|---|---|---|---|
| 3.051 | $OCH_3$ | N | $-CH_2OCH_2CH_2-$ | | $CH_3$ | $CHFCH_3$ | |
| 3.052 | $OCH_3$ | N | $-CH{=}CH-CH{=}CH-$ | | $CH_3$ | $CHFCH_3$ | |
| 3.053 | $OC_2H_5$ | N | $C_2H_5$ | $CH_3$ | $CH_3$ | $CHFCH_3$ | |
| 3.054 | $OCH_3$ | CH | H | H | $CH_3$ | $CHFCH_3$ | |
| 3.055 | $OC_2H_5$ | CH | $CH_3$ | H | $CH_3$ | $CHFCH_3$ | |
| 3.056 | $OCH_3$ | CH | H | $CH_3$ | $CH_3$ | $CHFCH_3$ | |
| 3.057 | $OC_2H_5$ | N | H | H | $C_2H_5$ | $CF_3$ | |
| 3.058 | $OCH_3$ | N | $CH_3$ | H | $C_2H_5$ | $CF_3$ | |
| 3.059 | $OCH_3$ | CH | H | H | $C_2H_5$ | $CF_3$ | |
| 3.060 | $OCH_3$ | N | $C_2H_5$ | H | $C_2H_5$ | $CHF_2$ | |
| 3.061 | $OC_2H_5$ | N | $n-C_3H_7$ | H | $C_2H_5$ | $CHF_2$ | |
| 3.062 | $OC_2H_5$ | N | $iso-C_3H_7$ | H | $C_2H_5$ | $CH_2F$ | |
| 3.063 | $OCH_3$ | N | $n-C_4H_9$ | H | $C_2H_5$ | $CH_2F$ | |
| 3.064 | $OCH_3$ | CH | $CH_3$ | H | $C_2H_5$ | $CH_2F$ | |
| 3.065 | $OCH_3$ | N | $-(CH_2)_3-$ | | $C_2H_5$ | $CHFCH_3$ | |
| 3.066 | $OCH_3$ | N | $-(CH_2)_4-$ | | $C_2H_5$ | $CHFCH_3$ | |
| 3.067 | $OC_2H_5$ | N | $-CH_2OCH_2CH_2-$ | | $C_2H_5$ | $CHFCH_3$ | |
| 3.068 | $OCH_3$ | N | $-CH{=}CH-CH{=}CH-$ | | $C_2H_5$ | $CHFCH_3$ | |
| 3.069 | $OCH_3$ | CH | H | $CH_3$ | $C_2H_5$ | $CHFCH_3$ | |
| 3.070 | $OC_2H_5$ | N | $C_2H_5$ | H | $n-C_3H_7$ | $CF_3$ | |
| 3.071 | $OCH_3$ | N | $n-C_3H_7$ | H | $n-C_3H_7$ | $CF_3$ | |
| 3.072 | $OCH_3$ | N | $iso-C_3H_7$ | H | $n-C_3H_7$ | $CHF_2$ | |
| 3.073 | $OC_2H_5$ | N | $n-C_4H_9$ | H | $n-C_3H_7$ | $CHF_2$ | |
| 3.074 | $OC_2H_5$ | N | $-(CH_2)_3-$ | | $n-C_3H_7$ | $CH_2F$ | |
| 3.075 | $OCH_3$ | N | $-(CH_2)_4-$ | | $n-C_3H_7$ | $CH_2F$ | |
| 3.076 | $OCH_3$ | N | H | H | $n-C_3H_7$ | $CHFCH_3$ | |
| 3.077 | $OC_2H_5$ | N | $CH_3$ | H | $n-C_3H_7$ | $CHFCH_3$ | |
| 3.078 | $OCH_3$ | N | $-CH_2OCH_2CH_2-$ | | $n-C_3H_7$ | $CHFCH_3$ | |
| 3.079 | $OC_2H_5$ | N | $-CH{=}CH-CH{=}CH-$ | | $n-C_3H_7$ | $CHFCH_3$ | |

| Verb. Nr. | A | X | $R^1$ | $R^2$ | $R^3$ | $R^4$ | Smp. [°C] phys. Daten |
|---|---|---|---|---|---|---|---|
| 3.080 | $OC_2H_5$ | N | iso-$C_3H_7$ | H | i-$C_3H_7$ | $CF_3$ | |
| 3.081 | $OCH_3$ | N | n-$C_4H_9$ | H | i-$C_3H_7$ | $CF_3$ | |
| 3.082 | $OC_2H_5$ | N | $-(CH_2)_3-$ | | i-$C_3H_7$ | $CHF_2$ | |
| 3.083 | $OCH_3$ | N | $-(CH_2)_4-$ | | i-$C_3H_7$ | $CHF_2$ | |
| 3.084 | $OCH_3$ | N | $-CH_2OCH_2CH_2-$ | | i-$C_3H_7$ | $CH_2F$ | |
| 3.085 | $OC_2H_5$ | N | -CH=CH-CH=CH- | | i-$C_3H_7$ | $CH_2F$ | |
| 3.086 | $OCH_3$ | N | H | H | i-$C_3H_7$ | $CHFCH_3$ | |
| 3.087 | $OCH_3$ | N | $CH_3$ | H | i-$C_3H_7$ | $CHFCH_3$ | |
| 3.088 | $OC_2H_5$ | N | $C_2H_5$ | H | i-$C_3H_7$ | $CHFCH_3$ | |
| 3.089 | $OCH_3$ | N | n-$C_3H_7$ | H | i-$C_3H_7$ | $CHFCH_3$ | |
| 3.090 | $OCH_3$ | N | $-(CH_2)_3-$ | | n-$C_4H_9$ | $CF_3$ | |
| 3.091 | $OC_2H_5$ | N | $-(CH_2)_4-$ | | n-$C_4H_9$ | $CF_3$ | |
| 3.092 | $OCH_3$ | N | $-CH_2OCH_2CH_2-$ | | n-$C_4H_9$ | $CHF_2$ | |
| 3.093 | $OC_2H_5$ | N | -CH=CH-CH=CH- | | n-$C_4H_9$ | $CHF_2$ | |
| 3.094 | $OCH_3$ | N | $C_2H_5$ | $CH_3$ | n-$C_4H_9$ | $CH_2F$ | |
| 3.095 | $OCH_3$ | CH | H | H | n-$C_4H_9$ | $CH_2F$ | |
| 3.096 | $OCH_3$ | N | H | H | n-$C_4H_9$ | $CHFCH_3$ | |
| 3.097 | $OCH_3$ | N | $CH_3$ | H | n-$C_4H_9$ | $CHFCH_3$ | |
| 3.098 | O-(n-$C_3H_7$) | N | H | H | $CH_3$ | $CF_3$ | |
| 3.099 | O-(iso-$C_3H_7$) | N | $CH_3$ | H | $CH_3$ | $CF_3$ | |
| 3.100 | O-(n-$C_3H_7$) | N | $C_2H_5$ | H | $CH_3$ | $CF_3$ | |
| 3.101 | O-(n-$C_3H_7$) | N | n-$C_3H_7$ | H | $CH_3$ | $CF_3$ | |
| 3.102 | O-(iso-$C_3H_7$) | N | iso-$C_3H_7$ | H | $CH_3$ | $CF_3$ | |
| 3.103 | O-(n-$C_3H_7$) | N | n-$C_4H_9$ | H | $CH_3$ | $CF_3$ | |
| 3.104 | O-(n-$C_3H_7$) | N | $-(CH_2)_3-$ | | $CH_3$ | $CF_3$ | |
| 3.105 | O-(n-$C_3H_7$) | N | $-(CH_2)_4-$ | | $CH_3$ | $CF_3$ | |
| 3.106 | O-(n-$C_3H_7$) | N | $-CH_2OCH_2CH_2-$ | | $CH_3$ | $CF_3$ | |
| 3.107 | O-(iso-$C_3H_7$) | N | -CH=CH-CH=CH- | | $CH_3$ | $CF_3$ | |

| Verb. Nr. | A | X | $R^1$ | $R^2$ | $R^3$ | $R^4$ | Smp. [°C] phys. Daten |
|---|---|---|---|---|---|---|---|
| 3.108 | O-(n-$C_3H_7$) | N | $C_2H_5$ | $CH_3$ | $CH_3$ | $CF_3$ | |
| 3.109 | O-(n-$C_3H_7$) | CH | H | H | $CH_3$ | $CF_3$ | |
| 3.110 | O-(n-$C_3H_7$) | CH | $CH_3$ | H | $CH_3$ | $CF_3$ | |
| 3.111 | O-(iso-$C_3H_7$) | CH | H | $CH_3$ | $CH_3$ | $CF_3$ | |
| 3.112 | O-(n-$C_3H_7$) | N | H | H | $CH_3$ | $CHF_2$ | |
| 3.113 | O-(n-$C_3H_7$) | N | $CH_3$ | H | $CH_3$ | $CHF_2$ | |
| 3.114 | O-(n-$C_3H_7$) | N | $C_2H_5$ | H | $CH_3$ | $CHF_2$ | |
| 3.115 | O-(n-$C_3H_7$) | N | n-$C_3H_7$ | H | $CH_3$ | $CHF_2$ | |
| 3.116 | O-(iso-$C_3H_7$) | N | iso-$C_3H_7$ | H | $CH_3$ | $CHF_2$ | |
| 3.117 | O-(n-$C_3H_7$) | N | n-$C_4H_9$ | H | $CH_3$ | $CHF_2$ | |
| 3.118 | O-(n-$C_3H_7$) | N | $-(CH_2)_3-$ | | $CH_3$ | $CHF_2$ | |
| 3.119 | O-(iso-$C_3H_7$) | N | $-(CH_2)_4-$ | | $CH_3$ | $CHF_2$ | |
| 3.120 | O-(n-$C_3H_7$) | N | $-CH_2OCH_2CH_2-$ | | $CH_3$ | $CHF_2$ | |
| 3.121 | O-(n-$C_3H_7$) | N | -CH=CH-CH=CH- | | $CH_3$ | $CHF_2$ | |
| 3.122 | O-(n-$C_3H_7$) | N | $C_2H_5$ | $CH_3$ | $CH_3$ | $CHF_2$ | |
| 3.123 | O-(n-$C_3H_7$) | CH | H | H | $CH_3$ | $CHF_2$ | |
| 3.124 | O-(iso-$C_3H_7$) | CH | $CH_3$ | H | $CH_3$ | $CHF_2$ | |
| 3.125 | O-(n-$C_3H_7$) | CH | H | $CH_3$ | $CH_3$ | $CHF_2$ | |
| 3.126 | O-(n-$C_3H_7$) | N | H | H | $CH_3$ | $CH_2F$ | |
| 3.127 | O-(n-$C_3H_7$) | N | $CH_3$ | H | $CH_3$ | $CH_2F$ | |
| 3.128 | O-(iso-$C_3H_7$) | N | $C_2H_5$ | H | $CH_3$ | $CH_2F$ | |
| 3.129 | O-(n-$C_3H_7$) | N | n-$C_3H_7$ | H | $CH_3$ | $CH_2F$ | |
| 3.130 | O-(n-$C_3H_7$) | N | iso-$C_3H_7$ | H | $CH_3$ | $CH_2F$ | |
| 3.131 | O-(n-$C_3H_7$) | N | n-$C_4H_9$ | H | $CH_3$ | $CH_2F$ | |
| 3.132 | O-(iso-$C_3H_7$) | N | $-(CH_2)_3-$ | | $CH_3$ | $CH_2F$ | |
| 3.133 | O-(n-$C_3H_7$) | N | $-(CH_2)_4-$ | | $CH_3$ | $CH_2F$ | |
| 3.134 | O-(n-$C_3H_7$) | N | $-CH_2OCH_2CH_2-$ | | $CH_3$ | $CH_2F$ | |
| 3.135 | O-(n-$C_3H_7$) | N | -CH=CH-CH=CH- | | $CH_3$ | $CH_2F$ | |
| 3.136 | O-(n-$C_3H_7$) | N | $C_2H_5$ | $CH_3$ | $CH_3$ | $CH_2F$ | |
| 3.137 | O-(iso-$C_3H_7$) | CH | H | H | $CH_3$ | $CH_2F$ | |

| Verb. Nr. | A | X | $R^1$ | $R^2$ | $R^3$ | $R^4$ | Smp. [°C] phys. Daten |
|---|---|---|---|---|---|---|---|
| 3.138 | O-(n-$C_3H_7$) | CH | $CH_3$ | H | $CH_3$ | $CH_2F$ | |
| 3.139 | O-(n-$C_3H_7$) | CH | H | $CH_3$ | $CH_3$ | $CH_2F$ | |
| 3.140 | O-(n-$C_3H_7$) | N | H | H | $CH_3$ | $CHFCH_3$ | |
| 3.141 | O-(iso-$C_3H_7$) | N | $CH_3$ | H | $CH_3$ | $CHFCH_3$ | |
| 3.142 | O-(n-$C_3H_7$) | N | $C_2H_5$ | H | $CH_3$ | $CHFCH_3$ | |
| 3.143 | O-(n-$C_3H_7$) | N | n-$C_3H_7$ | H | $CH_3$ | $CHFCH_3$ | |
| 3.144 | O-(iso-$C_3H_7$) | N | iso-$C_3H_7$ | H | $CH_3$ | $CHFCH_3$ | |
| 3.145 | O-(n-$C_3H_7$) | N | n-$C_4H_9$ | H | $CH_3$ | $CHFCH_3$ | |
| 3.146 | O-(n-$C_3H_7$) | N | $-(CH_2)_3-$ | | $CH_3$ | $CHFCH_3$ | |
| 3.147 | O-(iso-$C_3H_7$) | N | $-(CH_2)_4-$ | | $CH_3$ | $CHFCH_3$ | |
| 3.148 | O-(n-$C_3H_7$) | N | $-CH_2OCH_2CH_2-$ | | $CH_3$ | $CHFCH_3$ | |
| 3.149 | O-(n-$C_3H_7$) | N | -CH=CH-CH=CH- | | $CH_3$ | $CHFCH_3$ | |
| 3.150 | O-(iso-$C_3H_7$) | N | $C_2H_5$ | $CH_3$ | $CH_3$ | $CHFCH_3$ | |
| 3.151 | O-(n-$C_3H_7$) | CH | H | H | $CH_3$ | $CHFCH_3$ | |
| 3.152 | O-(n-$C_3H_7$) | CH | $CH_3$ | H | $CH_3$ | $CHFCH_3$ | |
| 3.153 | O-(n-$C_3H_7$) | CH | H | $CH_3$ | $CH_3$ | $CHFCH_3$ | |
| 3.154 | O-(iso-$C_3H_7$) | N | H | H | $C_2H_5$ | $CF_3$ | |
| 3.155 | O-(n-$C_3H_7$) | N | $CH_3$ | H | $C_2H_5$ | $CF_3$ | |
| 3.156 | O-(n-$C_3H_7$) | CH | H | H | $C_2H_5$ | $CF_3$ | |
| 3.157 | O-(n-$C_3H_7$) | N | $C_2H_5$ | H | $C_2H_5$ | $CHF_2$ | |
| 3.158 | O-(iso-$C_3H_7$) | N | n-$C_3H_7$ | H | $C_2H_5$ | $CHF_2$ | |
| 3.159 | O-(iso-$C_3H_7$) | N | iso-$C_3H_7$ | H | $C_2H_5$ | $CH_2F$ | |
| 3.160 | O-(n-$C_3H_7$) | N | n-$C_4H_9$ | H | $C_2H_5$ | $CH_2F$ | |
| 3.161 | O-(n-$C_3H_7$) | CH | $CH_3$ | H | $C_2H_5$ | $CH_2F$ | |
| 3.162 | O-(n-$C_3H_7$) | N | $-(CH_2)_3-$ | | $C_2H_5$ | $CHFCH_3$ | |
| 3.163 | O-(iso-$C_3H_7$) | N | $-(CH_2)_4-$ | | $C_2H_5$ | $CHFCH_3$ | |
| 3.164 | O-(n-$C_3H_7$) | N | $-CH_2OCH_2CH_2-$ | | $C_2H_5$ | $CHFCH_3$ | |
| 3.165 | O-(iso-$C_3H_7$) | N | -CH=CH-CH=CH- | | $C_2H_5$ | $CHFCH_3$ | |

| Verb. Nr. | A | X | $R^1$ | $R^2$ | $R^3$ | $R^4$ | Smp. [°C] phys. Daten |
|---|---|---|---|---|---|---|---|
| 3.166 | O-(n-$C_3H_7$) | CH | H | $CH_3$ | $C_2H_5$ | $CHFCH_3$ | |
| 3.167 | O-(iso-$C_3H_7$) | N | $C_2H_5$ | H | n-$C_3H_7$ | $CF_3$ | |
| 3.168 | O-(n-$C_3H_7$) | N | n-$C_3H_7$ | H | n-$C_3H_7$ | $CF_3$ | |
| 3.169 | O-(iso-$C_3H_7$) | N | iso-$C_3H_7$ | H | n-$C_3H_7$ | $CHF_2$ | |
| 3.170 | O-(n-$C_3H_7$) | N | n-$C_4H_9$ | H | n-$C_3H_7$ | $CHF_2$ | |
| 3.171 | O-(iso-$C_3H_7$) | N | $-(CH_2)_3-$ | | n-$C_3H_7$ | $CH_2F$ | |
| 3.172 | O-(n-$C_3H_7$) | N | $-(CH_2)_4-$ | | n-$C_3H_7$ | $CH_2F$ | |
| 3.173 | O-(n-$C_3H_7$) | N | H | H | n-$C_3H_7$ | $CHFCH_3$ | |
| 3.174 | O-(iso-$C_3H_7$) | N | $CH_3$ | H | n-$C_3H_7$ | $CHFCH_3$ | |
| 3.175 | O-(iso-$C_3H_7$) | N | $-CH_2OCH_2CH_2-$ | | n-$C_3H_7$ | $CHFCH_3$ | |
| 3.176 | O-(n-$C_3H_7$) | N | -CH=CH-CH=CH- | | n-$C_3H_7$ | $CHFCH_3$ | |
| 3.177 | O-(n-$C_3H_7$) | N | iso-$C_3H_7$ | H | i-$C_3H_7$ | $CF_3$ | |
| 3.178 | O-(iso-$C_3H_7$) | N | n-$C_4H_9$ | H | i-$C_3H_7$ | $CF_3$ | |
| 3.179 | O-(iso-$C_3H_7$) | N | $-(CH_2)_3-$ | | i-$C_3H_7$ | $CHF_2$ | |
| 3.180 | O-(n-$C_3H_7$) | N | $-(CH_2)_4-$ | | i-$C_3H_7$ | $CHF_2$ | |
| 3.181 | O-(iso-$C_3H_7$) | N | $-CH_2OCH_2CH_2-$ | | i-$C_3H_7$ | $CH_2F$ | |
| 3.182 | O-(n-$C_3H_7$) | N | -CH=CH-CH=CH- | | i-$C_3H_7$ | $CH_2F$ | |
| 3.183 | O-(n-$C_3H_7$) | N | H | H | i-$C_3H_7$ | $CHFCH_3$ | |
| 3.184 | O-(iso-$C_3H_7$) | N | $CH_3$ | H | i-$C_3H_7$ | $CHFCH_3$ | |
| 3.185 | O-(n-$C_3H_7$) | N | $C_2H_5$ | H | i-$C_3H_7$ | $CHFCH_3$ | |
| 3.186 | O-(iso-$C_3H_7$) | N | n-$C_3H_7$ | H | i-$C_3H_7$ | $CHFCH_3$ | |
| 3.187 | O-(iso-$C_3H_7$) | N | $-(CH_2)_3-$ | | n-$C_4H_9$ | $CF_3$ | |
| 3.188 | O-(n-$C_3H_7$) | N | $-(CH_2)_4-$ | | n-$C_4H_9$ | $CF_3$ | |
| 3.189 | O-(n-$C_3H_7$) | N | $-CH_2OCH_2CH_2-$ | | n-$C_4H_9$ | $CHF_2$ | |
| 3.190 | O-(iso-$C_3H_7$) | N | -CH=CH-CH=CH- | | n-$C_4H_9$ | $CHF_2$ | |
| 3.191 | O-(iso-$C_3H_7$) | N | $C_2H_5$ | $CH_3$ | n-$C_4H_9$ | $CH_2F$ | |
| 3.192 | O-(n-$C_3H_7$) | CH | H | H | n-$C_4H_9$ | $CH_2F$ | |
| 3.193 | O-(n-$C_3H_7$) | N | H | H | n-$C_4H_9$ | $CHFCH_3$ | |
| 3.194 | O-(iso-$C_3H_7$) | N | $CH_3$ | H | n-$C_4H_9$ | $CHFCH_3$ | |

| Verb. Nr. | A | X | $R^1$ | $R^2$ | $R^3$ | $R^4$ | Smp. [°C] phys. Daten |
|---|---|---|---|---|---|---|---|
| 3.195 | O-(n-$C_4H_9$) | N | H | H | $CH_3$ | $CF_3$ | |
| 3.196 | O-(n-$C_4H_9$) | N | $CH_3$ | H | $CH_3$ | $CF_3$ | |
| 3.197 | O-(sek.-$C_4H_9$) | N | $C_2H_5$ | H | $CH_3$ | $CF_3$ | |
| 3.198 | O-(n-$C_4H_9$) | N | n-$C_3H_7$ | H | $CH_3$ | $CF_3$ | |
| 3.199 | O-(sek.-$C_4H_9$) | N | iso-$C_3H_7$ | H | $CH_3$ | $CF_3$ | |
| 3.200 | O-(n-$C_4H_9$) | N | n-$C_4H_9$ | H | $CH_3$ | $CF_3$ | |
| 3.201 | O-(iso-$C_4H_9$) | N | $-(CH_2)_3-$ | | $CH_3$ | $CF_3$ | |
| 3.202 | O-(n-$C_4H_9$) | N | $-(CH_2)_4-$ | | $CH_3$ | $CF_3$ | |
| 3.203 | O-(tert-$C_4H_9$) | N | $-CH_2OCH_2CH_2-$ | | $CH_3$ | $CF_3$ | |
| 3.204 | O-(n-$C_4H_9$) | N | -CH=CH-CH=CH- | | $CH_3$ | $CF_3$ | |
| 3.205 | O-(n-$C_4H_9$) | N | $C_2H_5$ | $CH_3$ | $CH_3$ | $CF_3$ | |
| 3.206 | O-(n-$C_4H_9$) | CH | H | H | $CH_3$ | $CF_3$ | |
| 3.207 | O-(sek.-$C_4H_9$) | CH | $CH_3$ | H | $CH_3$ | $CF_3$ | |
| 3.208 | O-(n-$C_4H_9$) | CH | H | $CH_3$ | $CH_3$ | $CF_3$ | |
| 3.209 | O-(iso-$C_4H_9$) | N | H | H | $CH_3$ | $CHF_2$ | |
| 3.210 | O-(n-$C_4H_9$) | N | $CH_3$ | H | $CH_3$ | $CHF_2$ | |
| 3.211 | O-(n-$C_4H_9$) | N | $C_2H_5$ | H | $CH_3$ | $CHF_2$ | |
| 3.212 | O-(tert-$C_4H_9$) | N | n-$C_3H_7$ | H | $CH_3$ | $CHF_2$ | |
| 3.213 | O-(n-$C_4H_9$) | N | iso-$C_3H_7$ | H | $CH_3$ | $CHF_2$ | |
| 3.214 | O-(n-$C_4H_9$) | N | n-$C_4H_9$ | H | $CH_3$ | $CHF_2$ | |

| Verb. Nr. | A | X | $R^1$ | $R^2$ | $R^3$ | $R^4$ | Smp. [°C] phys. Daten |
|---|---|---|---|---|---|---|---|
| 3.215 | O-(n-$C_4H_9$) | N | $-(CH_2)_3-$ | | $CH_3$ | $CHF_2$ | |
| 3.216 | O-(iso-$C_4H_9$) | N | $-(CH_2)_4-$ | | $CH_3$ | $CHF_2$ | |
| 3.217 | O-(n-$C_4H_9$) | N | $-CH_2OCH_2CH_2-$ | | $CH_3$ | $CHF_2$ | |
| 3.218 | O-(n-$C_4H_9$) | N | -CH=CH-CH=CH- | | $CH_3$ | $CHF_2$ | |
| 3.219 | O-(tert-$C_4H_9$) | N | $C_2H_5$ | $CH_3$ | $CH_3$ | $CHF_2$ | |
| 3.220 | O-(sek.-$C_4H_9$) | CH | H | H | $CH_3$ | $CHF_2$ | |
| 3.221 | O-(sek.-$C_4H_9$) | CH | $CH_3$ | H | $CH_3$ | $CHF_2$ | |
| 3.222 | O-(n-$C_4H_9$) | CH | H | $CH_3$ | $CH_3$ | $CHF_2$ | |
| 3.223 | O-(n-$C_4H_9$) | N | H | H | $CH_3$ | $CH_2F$ | |
| 3.224 | O-(n-$C_4H_9$) | N | $CH_3$ | H | $CH_3$ | $CH_2F$ | |
| 3.225 | O-(iso-$C_4H_9$) | N | $C_2H_5$ | H | $CH_3$ | $CH_2F$ | |
| 3.226 | O-(sek.-$C_4H_9$) | N | n-$C_3H_7$ | H | $CH_3$ | $CH_2F$ | |
| 3.227 | O-(n-$C_4H_9$) | N | iso-$C_3H_7$ | H | $CH_3$ | $CH_2F$ | |
| 3.228 | O-(tert-$C_4H_9$) | N | n-$C_4H_9$ | H | $CH_3$ | $CH_2F$ | |
| 3.229 | O-(n-$C_4H_9$) | N | $-(CH_2)_3-$ | | $CH_3$ | $CH_2F$ | |
| 3.230 | O-(n-$C_4H_9$) | N | $-(CH_2)_4-$ | | $CH_3$ | $CH_2F$ | |
| 3.231 | O-(tert-$C_4H_9$) | N | $-CH_2OCH_2CH_2-$ | | $CH_3$ | $CH_2F$ | |
| 3.232 | O-(iso-$C_4H_9$) | N | -CH=CH-CH=CH- | | $CH_3$ | $CH_2F$ | |
| 3.233 | O-(sek.-$C_4H_9$) | N | $C_2H_5$ | $CH_3$ | $CH_3$ | $CH_2F$ | |
| 3.234 | O-(n-$C_4H_9$) | CH | H | H | $CH_3$ | $CH_2F$ | |
| 3.235 | O-(n-$C_4H_9$) | CH | $CH_3$ | H | $CH_3$ | $CH_2F$ | |
| 3.236 | O-(n-$C_4H_9$) | CH | H | $CH_3$ | $CH_3$ | $CH_2F$ | |
| 3.237 | O-(n-$C_4H_9$) | N | H | H | $CH_3$ | $CHFCH_3$ | |
| 3.238 | O-(tert-$C_4H_9$) | N | $CH_3$ | H | $CH_3$ | $CHFCH_3$ | |
| 3.239 | O-(iso-$C_4H_9$) | N | $C_2H_5$ | H | $CH_3$ | $CHFCH_3$ | |
| 3.240 | O-(sek.-$C_4H_9$) | N | n-$C_3H_7$ | H | $CH_3$ | $CHFCH_3$ | |
| 3.241 | O-(n-$C_4H_9$) | N | iso-$C_3H_7$ | H | $CH_3$ | $CHFCH_3$ | |
| 3.242 | O-(n-$C_4H_9$) | N | n-$C_4H_9$ | H | $CH_3$ | $CHFCH_3$ | |
| 3.243 | O-(sek.-$C_4H_9$) | N | $-(CH_2)_3-$ | | $CH_3$ | $CHFCH_3$ | |
| 3.244 | O-(iso-$C_4H_9$) | N | $-(CH_2)_4-$ | | $CH_3$ | $CHFCH_3$ | |

| Verb. Nr. | A | X | $R^1$ | $R^2$ | $R^3$ | $R^4$ | Smp. [°C] phys. Daten |
|---|---|---|---|---|---|---|---|
| 3.245 | O-(n-$C_4H_9$) | N | $-CH_2OCH_2CH_2-$ | | $CH_3$ | $CHFCH_3$ | |
| 3.246 | O-(n-$C_4H_9$) | N | -CH=CH-CH=CH- | | $CH_3$ | $CHFCH_3$ | |
| 3.247 | O-(sek.-$C_4H_9$) | N | $C_2H_5$ | $CH_3$ | $CH_3$ | $CHFCH_3$ | |
| 3.248 | O-(iso-$C_4H_9$) | CH | H | H | $CH_3$ | $CHFCH_3$ | |
| 3.249 | O-(n-$C_4H_9$) | CH | $CH_3$ | H | $CH_3$ | $CHFCH_3$ | |
| 3.250 | O-(n-$C_4H_9$) | CH | H | $CH_3$ | $CH_3$ | $CHFCH_3$ | |
| 3.251 | O-(n-$C_4H_9$) | N | H | H | $C_2H_5$ | $CF_3$ | |
| 3.252 | O-(tert-$C_4H_9$) | N | $CH_3$ | H | $C_2H_5$ | $CF_3$ | |
| 3.253 | O-(n-$C_4H_9$) | CH | H | H | $C_2H_5$ | $CF_3$ | |
| 3.254 | O-(n-$C_4H_9$) | N | $C_2H_5$ | H | $C_2H_5$ | $CHF_2$ | |
| 3.255 | O-(sek.-$C_4H_9$) | N | n-$C_3H_7$ | H | $C_2H_5$ | $CHF_2$ | |
| 3.256 | O-(n-$C_4H_9$) | N | iso-$C_3H_7$ | H | $C_2H_5$ | $CH_2F$ | |
| 3.257 | O-(n-$C_4H_9$) | N | n-$C_4H_9$ | H | $C_2H_5$ | $CH_2F$ | |
| 3.258 | O-(sek.-$C_4H_9$) | CH | $CH_3$ | H | $C_2H_5$ | $CH_2F$ | |
| 3.259 | O-(n-$C_4H_9$) | N | $-(CH_2)_3-$ | | $C_2H_5$ | $CHFCH_3$ | |
| 3.260 | O-(tert-$C_4H_9$) | N | $-(CH_2)_4-$ | | $C_2H_5$ | $CHFCH_3$ | |
| 3.261 | O-(iso-$C_4H_9$) | N | $-CH_2OCH_2CH_2-$ | | $C_2H_5$ | $CHFCH_3$ | |
| 3.262 | O-(n-$C_4H_9$) | N | -CH=CH-CH=CH- | | $C_2H_5$ | $CHFCH_3$ | |
| 3.263 | O-(n-$C_4H_9$) | CH | H | $CH_3$ | $C_2H_5$ | $CHFCH_3$ | |
| 3.264 | O-(n-$C_4H_9$) | N | $C_2H_5$ | H | n-$C_3H_7$ | $CF_3$ | |
| 3.265 | O-(n-$C_4H_9$) | N | n-$C_3H_7$ | H | n-$C_3H_7$ | $CF_3$ | |
| 3.266 | O-(sek.-$C_4H_9$) | N | iso-$C_3H_7$ | H | n-$C_3H_7$ | $CHF_2$ | |
| 3.267 | O-(n-$C_4H_9$) | N | n-$C_4H_9$ | H | n-$C_3H_7$ | $CHF_2$ | |
| 3.268 | O-(n-$C_4H_9$) | N | $-(CH_2)_3-$ | | n-$C_3H_7$ | $CH_2F$ | |
| 3.269 | O-(n-$C_4H_9$) | N | $-(CH_2)_4-$ | | n-$C_3H_7$ | $CH_2F$ | |
| 3.270 | O-(sek.-$C_4H_9$) | N | H | H | n-$C_3H_7$ | $CHFCH_3$ | |
| 3.271 | O-(n-$C_4H_9$) | N | $CH_3$ | H | n-$C_3H_7$ | $CHFCH_3$ | |
| 3.272 | O-(iso-$C_4H_9$) | N | $-CH_2OCH_2CH_2-$ | | n-$C_3H_7$ | $CHFCH_3$ | |
| 3.273 | O-(n-$C_4H_9$) | N | -CH=CH-CH=CH- | | n-$C_3H_7$ | $CHFCH_3$ | |

| Verb. Nr. | A | X | $R^1$ | $R^2$ | $R^3$ | $R^4$ | Smp. [°C] phys. Daten |
|---|---|---|---|---|---|---|---|
| 3.274 | $O-(n-C_4H_9)$ | N | $iso-C_3H_7$ | H | $i-C_3H_7$ | $CF_3$ | |
| 3.275 | $O-(n-C_4H_9)$ | N | $n-C_4H_9$ | H | $i-C_3H_7$ | $CF_3$ | |
| 3.276 | $O-(sek.-C_4H_9)$ | N | $-(CH_2)_3-$ | | $i-C_3H_7$ | $CHF_2$ | |
| 3.277 | $O-(n-C_4H_9)$ | N | $-(CH_2)_4-$ | | $i-C_3H_7$ | $CHF_2$ | |
| 3.278 | $O-(n-C_4H_9)$ | N | $-CH_2OCH_2CH_2-$ | | $i-C_3H_7$ | $CH_2F$ | |
| 3.279 | $O-(n-C_4H_9)$ | N | -CH=CH-CH=CH- | | $i-C_3H_7$ | $CH_2F$ | |
| 3.280 | $O-(n-C_4H_9)$ | N | H | H | $i-C_3H_7$ | $CHFCH_3$ | |
| 3.281 | $O-(sek.-C_4H_9)$ | N | $CH_3$ | H | $i-C_3H_7$ | $CHFCH_3$ | |
| 3.282 | $O-(iso-C_4H_9)$ | N | $C_2H_5$ | H | $i-C_3H_7$ | $CHFCH_3$ | |
| 3.283 | $O-(tert-C_4H_9)$ | N | $n-C_3H_7$ | H | $i-C_3H_7$ | $CHFCH_3$ | |
| 3.284 | $O-(n-C_4H_9)$ | N | $-(CH_2)_3-$ | | $n-C_4H_9$ | $CF_3$ | |
| 3.285 | $O-(n-C_4H_9)$ | N | $-(CH_2)_4-$ | | $n-C_4H_9$ | $CF_3$ | |
| 3.286 | $O-(n-C_4H_9)$ | N | $-CH_2OCH_2CH_2-$ | | $n-C_4H_9$ | $CHF_2$ | |
| 3.287 | $O-(sek.-C_4H_9)$ | N | -CH=CH-CH=CH- | | $n-C_4H_9$ | $CHF_2$ | |
| 3.288 | $O-(n-C_4H_9)$ | N | $C_2H_5$ | $CH_3$ | $n-C_4H_9$ | $CH_2F$ | |
| 3.289 | $O-(sek.-C_4H_9)$ | CH | H | H | $n-C_4H_9$ | $CH_2F$ | |
| 3.290 | $O-(iso-C_4H_9)$ | N | H | H | $n-C_4H_9$ | $CHFCH_3$ | |
| 3.291 | $O-(n-C_4H_9)$ | N | $CH_3$ | H | $n-C_4H_9$ | $CHFCH_3$ | |
| 3.292 | $O-C_2H_5$ | N | $C_2H_5$ | H | $CH_3$ | $CHFCH_3$ | 108-110 |
| 3.293 | $O-C_2H_5$ | N | H | H | $CH_3$ | $CHFCH_3$ | 108-110 |

Beispiel 7: Formulierungsbeispiele für Wirkstoffe der Formel I (% = Gewichtsprozent)

| a) Spritzpulver | a) | b) | c) |
|---|---|---|---|
| Wirkstoff gemäss Tabelle 1 oder 3 | 20 % | 60 % | 0,5 % |
| Na-Ligninsulfonat | 5 % | 5 % | 5 % |
| Na-Laurylsulfat | 3 % | - | - |
| Na-Diisobutylnaphthalinsulfonat | - | 6 % | 6 % |
| Octylphenolpolyethylenglykolether (7-8 Mol EO) | | 2 % | 2 % |
| Hochdisperse Kieselsäure | 5 % | 27 % | 27 % |
| Kaolin | 67 % | - | 59,5 % |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| b) Emulsions-Konzentrat | a) | b) |
|---|---|---|
| Wirkstoff gemäss Tabelle 1 oder 3 | 10 % | 1 % |
| Octylphenolpolyethylenglykolether (4-5 Mol EO) | 3 % | 3 % |
| Ca-Dodecylbenzolsulfonat | 3 % | 3 % |
| Ricinusölpolyglykolether (36 Mol EO) | 4 % | 4 % |
| Cyclohexanon | 30 % | 10 % |
| Xylolgemisch | 50 % | 79 % |

Aus diesem Konzentrat können durch Verdünnen mit Wasser Emulsionen jeder gewünschten Konzentration hergestellt werden.

| c) Stäubemittel | a) | b) |
|---|---|---|
| Wirkstoff gemäss Tabelle 1 oder 3 | 0,1 % | 1 % |
| Talkum | 99,9 % | - |
| Kaolin | - | 99 % |

Man erhält anwendungsfertige Stäubemittel, indem der Wirkstoff mit dem Träger vermischt und auf einer geeigneten Mühle vermahlen wird.

| d) Extruder Granulat | a) | b) |
|---|---|---|
| Wirkstoff gemäss Tabelle 1 oder 3 | 10 % | 1 % |
| Na-Ligninsulfonat | 2 % | 2 % |
| Carboxymethylcellulose | 1 % | 1 % |
| Kaolin | 87 % | 96 % |

Der Wirkstoff wird mit den Zusatzstoffen vermischt, vermahlen und mit Wasser angefeuchtet. Dieses Gemisch wird extrudiert und anschliessend im Luftstrom getrocknet.

| e) Umhüllungs-Granulat | |
|---|---|
| Wirkstoff gemäss Tabelle 1 oder 3 | 3 % |
| Polyethylenglykol (MG 200) | 3 % |
| Kaolin | 94 % |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

| f) Suspensions-Konzentrat | a) | b) |
|---|---|---|
| Wirkstoff gemäss Tabelle 1 oder 3 | 40 % | 5 % |
| Ethylenglykol Nonylphenolpolyethylenglykolether | 10 % | 10 % |
| (15 Mol EO) | 6 % | 1 % |
| Na-Ligninsulfonat | 10 % | 5 % |
| Carboxymethylcellulose | 1 % | 1 % |
| 37 %ige wässrige Formaldehyd-Lösung | 0,2 % | 0,2 % |
| Silikonöl in Form einer 75 %igen wässrigen Emulsion | 0,8 % | 0,8 % |
| Wasser | 32 % | 77 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Biologische Beispiele:

Beispiel 8: Pre-emergente Herbizid-Wirkung

Im Gewächshaus wird unmittelbar nach der Einsaat der Versuchspflanzen in Saatschalen die Erdoberfläche mit einer wässrigen Dispersion der Wirkstoffe, erhalten aus einem 25 %igen Emulsionskonzentrat behandelt. Es werden Konzentrationen von 4 kg Wirksubstanz/Hektar angewendet. Die Saatschalen werden im Gewächshaus bei 22-25° C und 50-70 % relativer Luftfeuchtigkeit gehalten und der Versuch nach 3 Wochen ausgewertet und die Resultate nach folgender Notenskala ausgewertet.

1 Pflanze hat nicht gekeimt oder ist eingegangen
2-3 sehr starke Schäden
4 starke Schäden
5 mittlere Schäden, die Pflanzen sind verkümmert
6 Schäden, die Pflanze kann regenerieren
7-8 leichte Schäden
9 normales Wachstum, wie das unbehandelter Pflanzen

In diesem Versuch zeigten die Verbindungen der Tabellen 1 bis 4 starke Herbizidwirkung.

Beispiel 9: Post-emergente Herbizid-Wirkung (Kontaktherbizid)

Eine Anzahl Unkräuter, sowohl monokotyle wie dikotyle, werden nach dem Auflaufen (im 4- bis 6-Blattstadium) mit einer wässrigen Wirkstoffdispersion in einer Dosierung von 4 kg Wirksubstanz pro Hektar auf die Pflanzen gespritzt und diese bei 24°-26° C und 45-60 % relativer Luftfeuchtigkeit gehalten. 15 Tage nach der Behandlung wird der Versuch nach der obigen Notenskala ausgewertet. Auch in diesem Versuch zeigen die Verbindungen der Tabellen 1 und 3 starke bis sehr starke Herbizidwirkung.

Beispiel 10: Herbizidwirkung vor dem Auflaufen der Pflanzen

Kunststofftöpfe werden mit expandiertem Vermiculit (Dichte: 0,135 $g/cm^3$, Wasserabsorptionsvermögen: 0,565 l/l) gefüllt. Nach dem Sättigen des nicht adsorptiven Vermiculits mit einer wässrigen Wirkstoffemulsion in deionisiertem Wasser, die die Wirkstoffe in einer Konzentration von 70,8 ppm enthält, werden Samen der folgenden Pflanzen auf die Oberfläche gesät: Kresse, Agrostis tenuis, Stellaria media und Digitaria sanguinalis. Die Versuchsgefässe werden anschliessend in einer Klimakammer bei 20° C, einer Beleuchtung von ca. 20 kLux und einer relativen Luftfeuchtigkeit von 70 % gehalten. Während der Keimphase von 4 bis 6 Tagen werden die Töpfe zur Erhöhung der örtlichen Luftfeuchtigkeit mit lichtdurchlässigem Material abgedeckt und mit deinoisiertem Wasser begossen. Nach dem 5. Tag wird dem Giesswasser 0,5 % eines handelsüblichen Flüssigdüngers (®Greenzit) zugesetzt. 12 Tage nach der Aussaat wird der Versuch ausgewertet und die Wirkung auf die Versuchspflanzen nach dem in Beispiel 8 gegebenen Massstab bewertet.

Die Resultate sind in der Tabelle 5 zusammengefasst:

Tabelle 5

| Verbindung No. | Kresse | Agrostis | Stellaria | Digitaria |
|---|---|---|---|---|
| 1.043 | 2 | 2 | 2 | 2 |
| 1.044 | 2 | 2 | 2 | 2 |
| 1.045 | 2 | 2 | 2 | 2 |
| 1.098 | 2 | 2 | 2 | 2 |

## Patentansprüche

**Patentansprüche für folgende Vertragsstaaten : AT, BE, CH, DE, FR, GB, GR, IT, LI, LU, NL, SE**

**1.** Verbindungen der Formel I

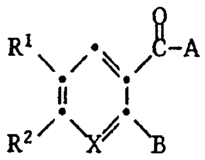

I,

worin

X — CH oder N

$R^1$ und $R^2$ — unabhängig voneinander Wasserstoff; ein gegebenenfalls ein- oder mehrfach durch Halogen, Nitro, Cyano, $C_1$-$C_4$-Alkoxy, $C_1$-$C_4$-Halogenalkoxy, $C_1$-$C_4$-Alkylthio oder $C_1$-$C_4$-Halogenalkylthio substituiertes $C_1$-$C_4$-Alkyl; oder

$R^1$ und $R^2$ — gemeinsam mit den Kohlenstoffatomen des Sechsringes, an welchen sie gebunden sind einen ungesättigten oder teilweise ungesättigten carbocyclischen oder bis zu zwei O oder S-Atome enthaltenden heterocyclischen 5 oder 6-Ring bilden; und

A — OH; $O^-M^+$; $C_1$-$C_4$-Alkoxy; $C_3$-$C_5$-Alkenyloxy; $C_3$-$C_5$-Alkinyloxy oder $C_1$-$C_4$-Alkoxy-$C_1$-$C_4$-alkoxy; und

$M^+$ — ein Kationenäquivalent eines Alkali- oder Erdalkalimetalls oder einer stickstoffhaltigen Base; und

B — ein Imidazolon der Formel

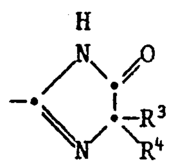

bilden; und

$R^3$ — $C_1$-$C_4$-Alkyl; und

$R^4$ — ein mit 1 bis 5 Fluoratomen substituierten Methyl- oder Ethylrest bedeutet,

sowie Salze von Verbindungen der Formel I mit Säuren, Basen und Komplexbildnern.

**2.** Verbindungen der Formel I, gemäss Anspruch 1, worin

X — CH oder N

$R^1$ und $R^2$ — unabhängig voneinander Wasserstoff oder $C_1$-$C_4$-Alkyl; oder

$R^1$ und $R_2$ — gemeinsam mit den Kohlenstoffatomen des Sechsringes an welchen sie gebunden sind einen ungesättigten oder teilweise ungesättigten carbocyclischen oder ein O oder S Atom enthaltenden heterocyclischen 5- oder 6-Ring bilden; und

A — OH; $O^{\ominus}M^{\oplus}$; $C_1$-$C_4$-Alkoxy; $C_3$-$C_5$-Alkenyloxy; $C_3$-$C_5$-Alkinyloxy; und

$M^{\oplus}$ — ein Kationenäquivalent eines Alkali- oder Erdalkalimetalls; oder einer stickstoffhaltigen Base; und

B — ein Imidazolon der Formel

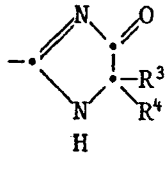

bilden; und

$R^3$ — $C_1$-$C_4$-Alkyl; und

$R^4$ — ein mit 1 bis 5 Fluoratomen substituierten Methyl- oder Ethylrest bedeutet.

**3.** 2-(5-Methyl-5-trifluormethyl-imidazol-4-on-2-yl)-isonicotinsäure,

2-(5-Methyl-5-trifluormethyl-imidazol-4-on-2-yl)-5-methyl-isonicotinsäure,
2-(5-Methyl-5-trifluormethyl-imidazol-4-on-2-yl)-5-ethyl-isonicotinsäure,
2-[5-(1-Fluorethyl)-5-methyl-imidazol-4-on-2-yl]-isonicotinsäure,
2-[5-(1-Fluorethyl)-5-methyl-imidazol-4-on-2-yl]-5-methyl-isonicotinsäure,
2-(5-Methyl-5-trifluormethyl-imidazol-4-on-2-yl)-chinolin-3-carbonsäure oder
2-[5-(1-Fluorethyl)-5-methyl-imidazol-4-on-2-yl]-5-ethyl-isonicotinsäure,
2-[5-Difluormethyl-5-methyl-imidazol-4-on-2-yl]-isonicotinsäure,
2-[5-Difluormethyl-5-methyl-imidazol-4-on-2-yl]-5-methyl-isonicotinsäure,
2-[5-Difluormethyl-5-methyl-imidazol-4-on-2-yl]-5-ethyl-isonicotinsäure,
2-[5-Difluormethyl-5-methylimidazol-4-on-2-yl]-5,6-dihydro-cyclopenta[b]pyridin-3-carbonsäure,
2-[5-Difluormethyl-5-methyl-imidazol-4-on-2-yl]-5,6,7,8-tetrahydrochinolin-3-carbonsäure,
2-[5-Difluormethyl-5-methyl-imidazol-4-on-2-yl]-chinolin-3-carbonsäure,
2-[5-(1-Fluorethyl)-5-methyl-imidazol-4-on-2-yl]-5,6-dihydro-cyclopenta[b]pyridin-3-carbonsäure,
2-[5-(1-Fluorethyl)-5-methyl-imidazol-4-on-2-yl]-5,6,7,8-tetrahydrochinolin-3-carbonsäure,
2-[5-(1-Fluorethyl)-5-methyl-imidazol-4-on-2-yl]-isonicotinsäuremethylester,
2-[5-(1-Fluorethyl)-5-methyl-imidazol-4-on-2-yl]-isonicotinsäureethylester oder
2-[5-(1-Fluorethyl)-5-methyl-imidazol-4-on-2-yl]-5-ethyl-isonicotinsäureethylester gemäss Anspruch 1 oder 2.

**4.** Verfahren zur Herstellung von Verbindungen der Formel Ia,

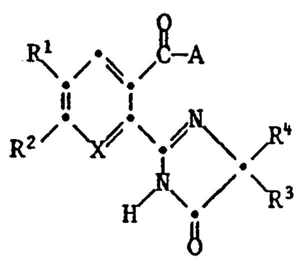

Ia

worin die Reste $R^1$ bis $R^4$, X und A wie in Anspruch 1 oder 2 definiert sind, dadurch gekennzeichnet, dass man
einen Diester der Formel IV

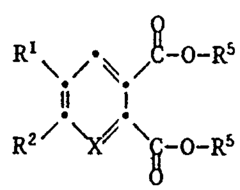

IV,

worin X, $R^1$ und $R^2$ wie zuvor definiert sind und die Reste $R^5$ unabhängig voneinander für $C_1$-$C_6$-Alkyl stehen mit einem $\alpha$-Aminosäureamid der Formel II

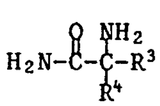

II

in Gegenwart einer mindestens äquimolaren Menge einer Base umsetzt und das Reaktionsgemisch anschliessend hydrolytisch aufarbeitet.

**5.** Verfahren zur Herstellung von Salzen der Formel Ia'

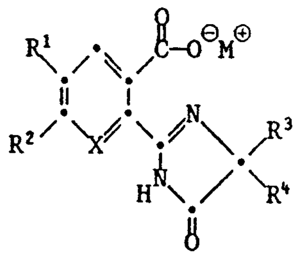

Ia'

gemäss Anspruch 1 oder 2,
worin X, $R^1$, $R^2$, $R^3$ und $R^4$ wie zuvor definiert sind und $M^{\oplus}$ für ein Kationenäquivalent eines Alkali- oder Erdalkalimetalls oder einer stickstoffhaltigen Base steht,
dadurch gekennzeichnet, dass man

a) eine Verbindung der Formel Ia

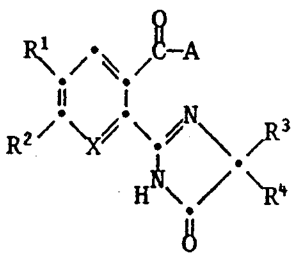

Ia,

in der die Reste X, $R^1$, $R^2$, $R^3$ und $R^4$ wie zuvor definiert sind und A für OH steht mit einer äquivalenten Menge einer Verbindung der Formel VII

$OH^{\ominus}M^{\oplus}$ VII,

in der $M^{\oplus}$ für ein Kationenäquivalent eines Alkali- oder Erdalkalimetalls oder eine quaternäre stickstoffhaltige Base steht, umsetzt, oder

b) eine Verbindung der Formel Ia, in der A für OH steht mit einem Alkali- oder Erdalkalimetall oder einem Alkali oder Erdalkalisalz oder der stickstoffhaltigen Base direkt umsetzt.

**6.** Herbizides Mittel, enthaltend eine Verbindung der Formel I gemäss einem der Ansprüche 1 bis 3 neben weiteren Hilfs- und/oder Trägerstoffen.

**7.** Verfahren zur Bekämpfung unerwünschten Pflanzenwuchses, dadurch gekennzeichnet, dass man eine Verbindung der Formel I gemäss Anspruch 1 bis 3 oder ein Mittel gemäss Anspruch 6 auf den Lebensraum der zu bekämpfenden Pflanze einwirken lässt.

**8.** Saatgut gekennzeichnet durch einen herbizid wirksamen Gehalt an einer Verbindung der Formel I gemäss einem der Ansprüche 1 bis 3.

**9.** $\alpha$-Aminosäureamide der Formel II

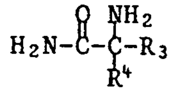

II,

worin

$R^3$ $C_1$-$C_4$-Alkyl und

$R^4$ einen mit 1 bis 5-Fluoratomen substituierten Methyl- oder Ethylrest bedeutet.

**10.** Verfahren zur Herstellung von α-Aminosäureamiden der Formel II gemäss Anspruch 9, dadurch gekennzeichnet, dass man

a) ein Oxazolin-5-on der Formel XI, worin $R^3$ und $R^4$ wie zuvor definiert sind, mit $NH_3$ zur Verbindung XII umsetzt und aus Verbindung XII die Trifluoracetylschutzgruppen abspaltet

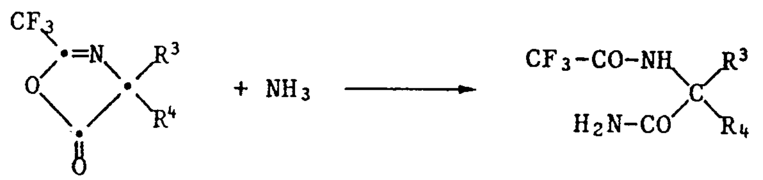

XII

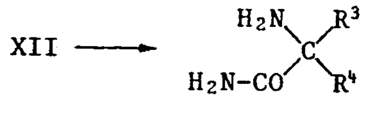

oder

b) ein Keton XIII, worin die Reste $R^3$ und $R^4$ wie zuvor definiert sind, $NH_3$ und Cyanid unter den Bedingungen der Strecker-Synthese zu einer Verbindung XIV addiert und Verbindung XIV ausschliesslich zu II hydrolisiert

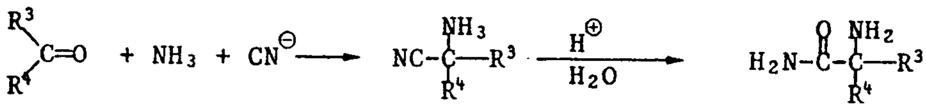


XIII XIV II

**Patentansprüche für folgenden Vertragsstaat : ES**

**1.** Verfahren zur Herstellung von Verbindungen der Formel Ia

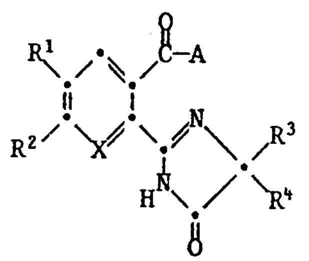

Ia,

worin

| | |
|---|---|
| X | CH oder N |
| $R^1$ und $R^2$ | unabhängig voneinander Wasserstoff; ein gegebenenfalls ein- oder mehrfach durch Halogen, Nitro, Cyano, $C_1$-$C_4$-Alkoxy, $C_1$-$C_4$-Halogenalkoxy, $C_1$-$C_4$-Alkylthio oder $C_1$-$C_4$-Halogenalkylthio substituiertes $C_1$-$C_4$-Alkyl; oder |
| $R^1$ und $R^2$ | gemeinsam mit den Kohlenstoffatomen des Sechsringes, an welchen sie gebunden sind einen ungesättigten oder teilweise ungesättigten carbocyclischen oder bis zu zwei 0 oder S-Atome enthaltenden heterocyclischen 5 oder 6-Ring bilden; und |
| A | OH; $O^-M^+$; $C_1$-$C_4$-Alkoxy; $C_3$-$C_5$-Alkenyloxy; $C_3$-$C_5$-Alkinyloxy oder $C_1$-$C_4$-Alkoxy-$C_1$-$C_4$-alkoxy; und |
| $M^+$ | ein Kationenäquivalent eines Alkali- oder Erdalkalimetalls oder einer stickstoffhaltigen Base; und |

$R^3$ $C_1$-$C_4$-Alkyl; und
$R^4$ ein mit 1 bis 5 Fluoratomen substituierten Methyl- oder Ethylrest bedeutet,
dadurch gekennzeichnet, dass man
einen Diester der Formel IV

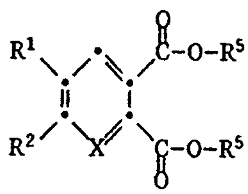

IV,

worin X, $R^1$ und $R^2$ wie zuvor definiert sind und die Reste $R^5$ unabhängig voneinander für $C_1$-$C_6$-Alkyl stehen mit einem $\alpha$-Aminosäureamid der Formel II

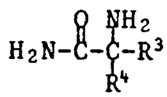

II

in Gegenwart einer mindestens äquimolaren Menge einer Base umsetzt und das Reaktionsgemisch anschliessend hydrolytisch aufarbeitet.

**2.** Verfahren zur Herstellung von Salzen der Formel Ia'

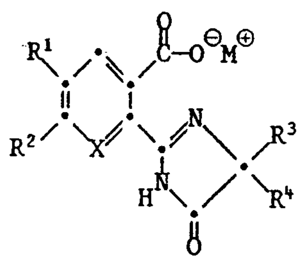

Ia'

worin X, $R^1$, $R^2$, $R^3$ und $R^4$ wie unter Anspruch 1 definiert sind und $M^{\oplus}$ für ein Kationenäquivalent eines Alkali- oder Erdalkalimetalls oder einer stickstoffhaltigen Base steht,
dadurch gekennzeichnet, dass man
a) eine Verbindung der Formel Ia

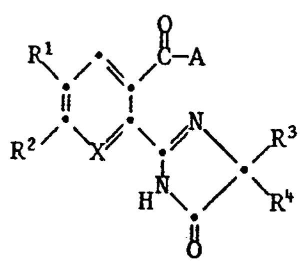

Ia,

in der die Reste X, $R^1$, $R^2$, $R^3$ und $R^4$ wie zuvor definiert sind und A für OH steht mit einer äquivalenten Menge einer Verbindung der Formel VII

$OH^{\ominus}M^{\oplus}$ VII,

in der $M^{\oplus}$ für ein Kationenäquivalent eines Alkali- oder Erdalkalimetalls oder eine quaternäre

stickstoffhaltige Base steht, umsetzt, oder

b) eine Verbindung der Formel Ia, in der A für OH steht mit einem Alkali- oder Erdalkalimetall oder einem Alkali oder Erdalkalisalz oder der stickstoffhaltigen Base direkt umsetzt.

**3.** Verfahren zur Bekämpfung unerwünschten Pflanzenwuchses, dadurch gekennzeichnet, dass man eine Verbindung der Formel I

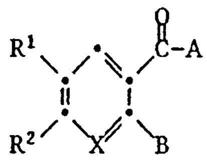

I,

worin

| | |
|---|---|
| X | CH oder N |
| $R^1$ und $R^2$ | unabhängig voneinander Wasserstoff; ein gegebenenfalls ein- oder mehrfach durch Halogen, Nitro, Cyano, $C_1$-$C_4$-Alkoxy, $C_1$-$C_4$-Halogenalkoxy, $C_1$-$C_4$-Alkylthio oder $C_1$-$C_4$-Halogenalkylthio substituiertes $C_1$-$C_4$-Alkyl; oder |
| $R^1$ und $R^2$ | gemeinsam mit den Kohlenstoffatomen des Sechsringes, an welchen sie gebunden sind einen ungesättigten oder teilweise ungesättigten carbocyclischen oder bis zu zwei O oder S-Atome enthaltenden heterocyclischen 5 oder 6-Ring bilden; und |
| A | OH; $O^-M^+$; $C_1$-$C_4$-Alkoxy; $C_3$-$C_5$-Alkenyloxy; $C_3$-$C_5$-Alkinyloxy oder $C_1$-$C_4$-Alkoxy-$C_1$-$C_4$-alkoxy; und |
| $M^+$ | ein Kationenäquivalent eines Alkali- oder Erdalkalimetalls oder einer stickstoffhaltigen Base; und |
| B | ein Imidazolon der Formel |

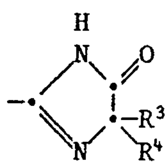

| | |
|---|---|
| | bilden; und |
| $R^3$ | $C_1$-$C_4$-Alkyl; und |
| $R^4$ | ein mit 1 bis 5 Fluoratomen substituierten Methyl- oder Ethylrest bedeutet, |

auf den Lebensraum der zu bekämpfenden Pflanzen einwirken lässt.

**4.** Verfahren gemäss Anspruch 3 zur Bekämpfung von Unkräutern oder Gräsern von Nutzpflanzkulturen.

**5.** Verfahren zur Herstellung eines herbiziden Mittels, dadurch gekennzeichnet, dass man eine Verbindung der Formel I

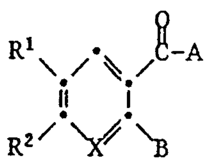

I,

worin die Reste $R^1$, $R^2$, X, A und B wie in Anspruch 3 definiert sind mit Hilfs- und/oder Trägerstoffen mischt.

**6.** Verfahren zur Saatgutbeizung, dadurch gekennzeichnet, dass man eine herbizid wirksame Menge einer

Verbindung der Formel I

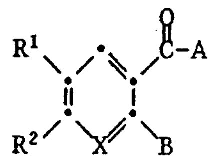

I,

worin die Reste $R^1$, $R^2$, X, A und B wie in Anspruch 3 definiert sind, dadurch gekennzeichnet, dass man eine herbizid wirksame Menge der Verbindung der Formel I auf das Saatgut aufbringt.

**7.** Verfahren zur Herstellung von α-Aminosäureamiden der Formel II

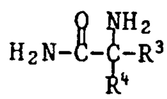

II,

worin

$R^3$ $C_1$-$C_4$-Alkyl; und

$R^4$ ein mit 1 bis 5 Fluoratomen substituierten Methyl- oder Ethylrest bedeutet,

dadurch gekennzeichnet, dass man

a) ein Oxazolin-5-on der Formel XI, worin $R^3$ und $R^4$ wie zuvor definiert sind, mit $NH_3$ zur Verbindung XII umsetzt und aus Verbindung XII die Trifluoracetylschutzgruppen abspaltet

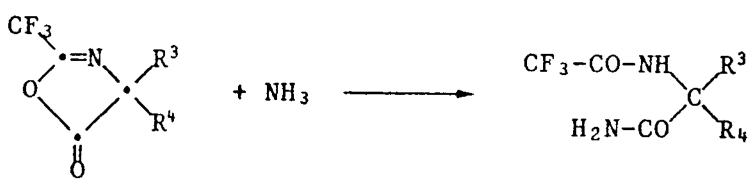

XII

XII 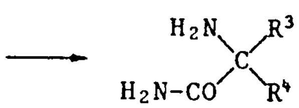

oder

b) ein Keton XIII, worin die Reste $R^3$ und $R^4$ wie zuvor definiert sind, $NH_3$ und Cyanid unter den Bedingungen der Strecker-Synthese zu einer Verbindung XIV addiert und Verbindung XIV ausschliesslich zu II hydrolisiert

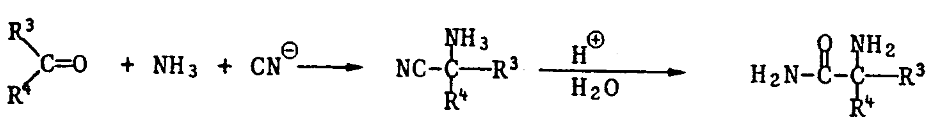


XIII XIV II

## Claims

**Claims for the following Contracting States : AT, BE, CH, DE, FR, GB, GR, IT, LI, LU, NL, SE**

**1.** A compound of formula I

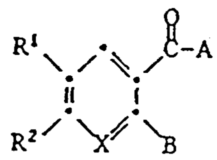

I

in which X represents CH or N, $R^1$ and $R^2$ independently of one another represent hydrogen; or $C_1$-$C_4$alkyl that is unsubstituted or mono- or poly-substituted by halogen, nitro, cyano, $C_1$-$C_4$alkoxy, $C_1$-$C_4$haloalkoxy, $C_1$-$C_4$alkylthio or by $C_1$-$C_4$haloalkylthio; or $R^1$ and $R^2$, together with the carbon atoms of the six-membered ring to which they are bonded, form an unsaturated or partially unsaturated carbocyclic 5- or 6-membered ring or an unsaturated or partially unsaturated heterocyclic 5- or 6-membered ring containing up to two O or S atoms; and A represents OH; $O^{\ominus}M^{\oplus}$; $C_1$-$C_4$alkoxy; $C_3$-$C_5$alkenyloxy;$C_3$-$C_5$alkynyloxy or $C_1$-$C_4$alkoxy-$C_1$-$C_4$alkoxy; and $M^{\oplus}$ represents a cation equivalent of an alkali metal or alkaline earth metal or of a nitrogen-containing base; and B represents an imidazolone of the formula

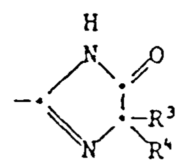

;

and $R^3$ represents $C_1$-$C_4$alkyl; and $R^4$ represents a methyl or ethyl radical substituted by from 1 to 5 fluorine atoms,
or a salt of a compound of formula I with an acid, a base or a complex-former.

**2.** A compound of formula I according to claim 1, in which X represents CH or N, $R^1$ and $R^2$ independently of one another represent hydrogen or $C_1$-$C_4$alkyl; or $R^1$ and $R^2$, together with the carbon atoms of the six-membered ring to which they are bonded, form an unsaturated or partially unsaturated carbocyclic 5- or 6-membered ring or an unsaturated or partially unsaturated heterocyclic 5- or 6-membered ring containing one O or S atom; and A represents OH; $O^{\ominus}M^{\oplus}$; $C_1$-$C_4$alkoxy; $C_3$-$C_5$alkenyloxy or $C_3$-$C_5$alkynyloxy; and $M^{\oplus}$ represents a cation equivalent of an alkali metal or alkaline earth metal; or of a nitrogen-containing base; and B represents an imidazolone of the formula

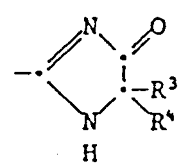

;

and $R^3$ represents $C_1$-$C_4$alkyl; and $R^4$ represents a methyl or ethyl radical substituted by from 1 to 5 fluorine atoms.

**3.** 2-(5-Methyl-5-trifluoromethylimidazol-4-on-2-yl)-isonicotinic acid,
2-(5-methyl-5-trifluoromethylimidazol-4-on-2-yl)-5-methylisonicotinic acid,
2-(5-methyl-5-trifluoromethylimidazol-4-on-2-yl)-5-ethylisonicotinic acid,
2-[5-(1-fluoroethyl)-5-methylimidazol-4-on-2-yl]-isonicotinic acid,
2-[5-(1-fluoroethyl)-5-methylimidazol-4-on-2-yl]-5-methylisonicotinic acid,
2-(5-methyl-5-trifluoromethylimidazol-4-on-2-yl)-quinoline-3-carboxylic acid, or
2-[5-(1-fluoroethyl)-5-methylimidazol-4-on-2-yl]-5-ethylisonicotinic acid,
2-[5-difluoromethyl-5-methylimidazol-4-on-2-yl]-isonicotinic acid,
2-[5-difluoromethyl-5-methylimidazol-4-on-2-yl]-5-methylisonicotinic acid,
2-[5-difluoromethyl-5-methylimidazol-4-on-2-yl]-5-ethylisonicotinic acid,
2-[5-difluoromethyl-5-methylimidazol-4-on-2-yl]-5,6-dihydrocyclopenta[b]pyridine-3-carboxylic acid,

2-[5-difluoromethyl-5-methylimidazol-4-on-2-yl]-5,6,7,8-tetrahydroquinoline-3-carboxylic acid,
2-[5-difluoromethyl-5-methylimidazol-4-on-2-yl]-quinoline-3-carboxylic acid,
2-[5-(1-fluoroethyl)-5-methylimidazol-4-on-2-yl]-5,6-dihydrocyclopenta[b]pyridine-3-carboxylic acid,
2-[5-(1-fluoroethyl)-5-methylimidazol-4-on-2-yl]-5,6,7,8-tetrahydroquinoline-3-carboxylic acid,
2-[5-(1-fluoroethyl)-5-methylimidazol-4-on-2-yl]-isonicotinic acid methyl ester,
2-[5-(1-fluoroethyl)-5-methylimidazol-4-on-2-yl]-isonicotinic acid ethyl ester
or
2-[5-(1-fluoroethyl)-5-methylimidazol-4-on-2-yl]-5-ethylisonicotinic acid ethyl ester according to either claim 1 or claim 2.

**4.** A process for the preparation of a compound of formula Ia

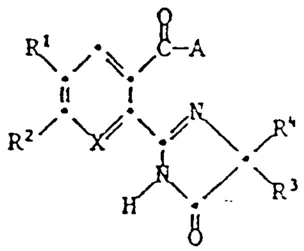

Ia

in which the radicals $R^1$ to $R^4$, X and A are as defined in either claim 1 or claim 2, characterised in that a diester of formula IV

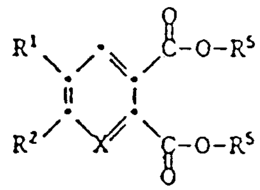

IV

in which X, $R^1$ and $R^2$ are as defined above and the radicals $R^5$ independently of one another represent $C_1$-$C_6$alkyl, is reacted with an $\alpha$-amino acid amide of formula II

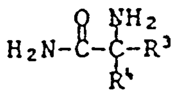

II

in the presence of at least an equimolar amount of a base, and the reaction mixture is then worked up by hydrolysis.

**5.** A process for the preparation of a salt of formula Ia'

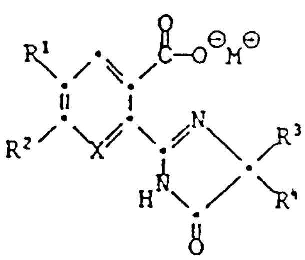

Ia'

according to either claim 1 or claim 2,
in which X, $R^1$, $R^2$, $R^3$ and $R^4$ are as defined above and $M^{\oplus}$ represents a cation equivalent of an alkali metal or alkaline earth metal or of a nitrogen-containing base,
characterised in that

a) a compound of formula Ia

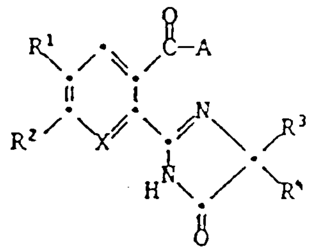

Ia

in which the radicals X, $R^1$, $R^2$, $R^3$ and $R^4$ are as defined above and A represents OH, is reacted with an equivalent amount of a compound of formula VII

$OH^{\ominus}M^{\oplus}$ VII

in which $M^{\oplus}$ represents a cation equivalent of an alkali metal or alkaline earth metal or a quaternary nitrogen-containing base, or

b) a compound of formula Ia in which A represents OH is reacted directly with an alkali metal or alkaline earth metal or with an alkali metal salt or alkaline earth metal salt or the nitrogen-containing base.

**6.** A herbicidal composition comprising a compound of formula I according to any one of claims 1 to 3 together with other adjuvants and/or carriers.

**7.** A method of controlling undesired plant growth, characterised in that a compound of formula I according to any one of claims 1 to 3 or a composition according to claim 6 is allowed to act on the locus of the plant to be controlled.

**8.** Seeds characterised in that they contain a herbicidally effective amount of a compound of formula I according to any one of claims 1 to 3.

**9.** An $\alpha$-amino acid amide of formula II

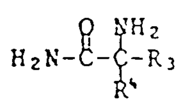

II

in which $R^3$ represents $C_1$-$C_4$alkyl and $R^4$ represents a methyl or ethyl radical substituted by from 1 to 5 fluorine atoms.

**10.** A process for the preparation of an $\alpha$-amino acid amide of formula II according to claim 9, characterised in that

a) an oxazolin-5-one of formula XI, in which $R^3$ and $R^4$ are as defined above, is reacted with $NH_3$ to give the compound XII, and the trifluoroacetyl protecting group is removed from the compound XII

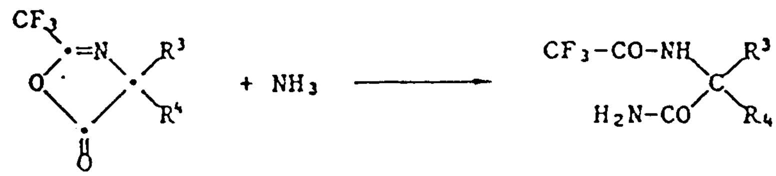

XII

XII 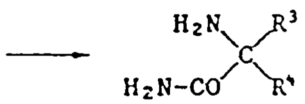

or

b) a ketone XIII, in which the radicals $R^3$ and $R^4$ are as defined above, $NH_3$ and cyanide are added together under the conditions of the Strecker synthesis to give a compound XIV, and the compound XIV is hydrolysed exclusively to give II

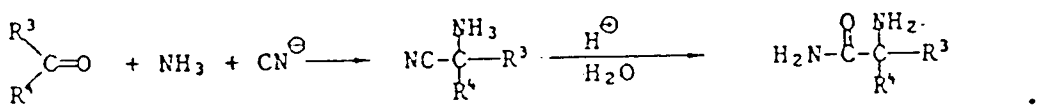


XIII XIV II

**Claims for the following Contracting State : ES**

1. A process for the preparation of a compound of formula Ia

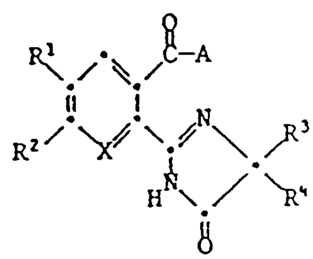

Ia

in which X represents CH or N, $R^1$ and $R^2$ independently of one another represent hydrogen; or $C_1$-$C_4$alkyl that is unsubstituted or mono- or poly-substituted by halogen, nitro, cyano, $C_1$-$C_4$alkoxy, $C_1$-$C_4$haloalkoxy, $C_1$-$C_4$alkylthio or by $C_1$-$C_4$haloalkylthio; or $R^1$ and $R^2$, together with the carbon atoms of the six-membered ring to which they are bonded, form an unsaturated or partially unsaturated carbocyclic 5- or 6-membered ring or an unsaturated or partially unsaturated heterocyclic 5- or 6-membered ring containing up to two O or S atoms; and A represents OH; $O^{\ominus}M^{\oplus}$; $C_1$-$C_4$alkoxy; $C_3$-$C_5$alkenyloxy;$C_3$-$C_5$alkynyloxy or $C_1$-$C_4$alkoxy-$C_1$-$C_4$alkoxy; and $M^{\oplus}$ represents a cation equivalent of an alkali metal or alkaline earth metal or of a nitrogen-containing base; and $R^3$ represents $C_1$-$C_4$alkyl; and $R^4$ represents a methyl or ethyl radical substituted by from 1 to 5 fluorine atoms,
characterised in that a diester of formula IV

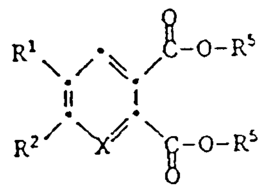

IV

in which X, $R^1$ and $R^2$ are as defined above and the radicals $R^5$ independently of one another represent $C_1$-$C_6$alkyl, is reacted with an $\alpha$-amino acid amide of formula II

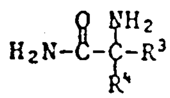

II

in the presence of at least an equimolar amount of a base, and the reaction mixture is then worked up by hydrolysis.

**2.** A process for the preparation of a salt of formula Ia'

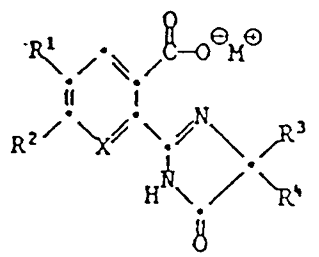

Ia'

in which X, $R^1$, $R^2$, $R^3$ and $R^4$ are as defined under claim 1 and $M^{\oplus}$ represents a cation equivalent of an alkali metal or alkaline earth metal or of a nitrogen-containing base,
characterised in that
a) a compound of formula Ia

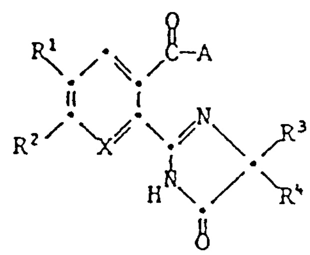

Ia

in which the radicals X, $R^1$, $R^2$, $R^3$ and $R^4$ are as defined above and A represents OH, is reacted with an equivalent amount of a compound of formula VII

$OH^{\ominus}M^{\oplus}$ VII

in which $M^{\oplus}$ represents a cation equivalent of an alkali metal or alkaline earth metal or a quaternary nitrogen-containing base, or
b) a compound of formula Ia in which A represents OH is reacted directly with an alkali metal or alkaline earth metal or with an alkali metal salt or alkaline earth metal salt or the nitrogen-containing base.

3. A method of controlling undesired plant growth, characterised in that a compound of formula I

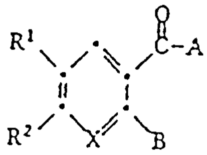

I

in which X represents CH or N, $R^1$ and $R^2$ independently of one another represent hydrogen; or $C_1$-$C_4$alkyl that is unsubstituted or mono- or poly-substituted by halogen, nitro, cyano, $C_1$-$C_4$alkoxy, $C_1$-$C_4$haloalkoxy, $C_1$-$C_4$alkylthio or by $C_1$-$C_4$haloalkylthio; or $R^1$ and $R^2$, together with the carbon atoms of the six-membered ring to which they are bonded, form an unsaturated or partially unsaturated carbocyclic 5- or 6-membered ring or an unsaturated or partially unsaturated heterocyclic 5- or 6-membered ring containing up to two O or S atoms; and A represents OH; $O^{\ominus}M^{\oplus}$; $C_1$-$C_4$alkoxy; $C_3$-$C_5$alkenyloxy;$C_3$-$C_5$alkynyloxy or $C_1$-$C_4$alkoxy-$C_1$-$C_4$alkoxy; and $M^{\oplus}$ represents a cation equivalent of an alkali metal or alkaline earth metal or of a nitrogen-containing base; and B represents an imidazolone of the formula

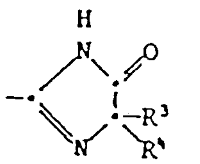

;

and $R^3$ represents $C_1$-$C_4$alkyl; and $R^4$ represents a methyl or ethyl radical substituted by from 1 to 5 fluorine atoms,
is allowed to act on the locus of the plants to be controlled.

4. A method according to claim 3 of controlling weeds or grasses in crops of useful plants.

5. A process for the preparation of a herbicidal composition, characterised in that a compound of formula I

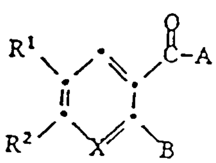

I

in which the radicals $R^1$, $R^2$, X, A and B are as defined in claim 3, is mixed with adjuvants and/or carriers.

6. A method of dressing seeds, characterised in that a herbicidally effective amount of a compound of formula I

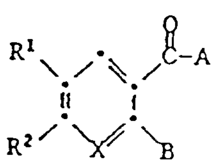

I

in which the radicals $R^1$, $R^2$, X, A and B are as defined in claim 3, is applied to the seeds.

7. A process for the preparation of an α-amino acid amide of formula II

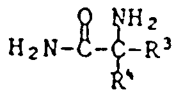

II

in which $R^3$ represents $C_1$-$C_4$alkyl and $R^4$ represents a methyl or ethyl radical substituted by from 1 to 5 fluorine atoms,
characterised in that

a) an oxazolin-5-one of formula XI, in which $R^3$ and $R^4$ are as defined above, is reacted with $NH_3$ to give the compound XII, and the trifluoroacetyl protecting group is removed from the compound XII

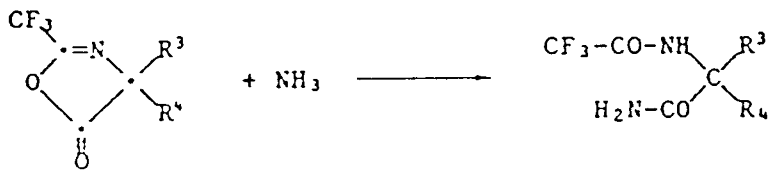


XII

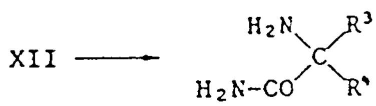

or

b) a ketone XIII, in which the radicals $R^3$ and $R^4$ are as defined above, $NH_3$ and cyanide are added together under the conditions of the Strecker synthesis to give a compound XIV, and the compound XIV is hydrolysed exclusively to give II

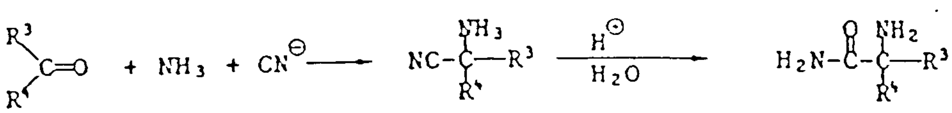


XIII XIV II

**Revendications**

**Revendications pour les Etats contractants suivants : AT, BE, CH, DE, FR, GB, GR, IT, LI, LU, NL, SE**

1. Composés de formule I

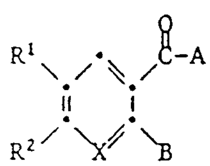

I,

où

X représente CH ou N,

$R^1$ et $R^2$ représentent, indépendamment l'un de l'autre, l'hydrogène; un alkyle en $C_1$-$C_4$ éventuellement substitué, une ou plusieurs fois par un halogène, par le nitro, par le cyano, par un alcoxy en $C_1$-$C_4$, par un halogénoalcoxy en $C_1$-$C_4$, par un alkyle en $C_1$-$C_4$ thio

ou par un halogénoalkyle en $C_1$-$C_4$ thio ou

$R^1$ et $R^2$ forment ensemble avec les atomes de carbone du noyau hexagonal auxquels ils sont liés un noyau carbocyclique pentagonal ou hexagonal insaturé ou en partie insaturé ou un noyau hétérocyclique pentagonal ou hexagonal contenant jusqu'à deux atomes de O ou de S et

A représente OH; $O^-M^+$ ; un alcoxy en $C_1$-$C_4$, un alcényle en $C_3$-$C_5$ oxy, un alcynyle en $C_3$-$C_5$ oxy ou un alcoxy en $C_1$-$C_4$ alcoxy en $C_1$-$C_4$ et

$M^+$ un équivalent cation d'un métal alcalin ou alcalino-terreux ou d'une base azotée et

B une imidazolone de formule

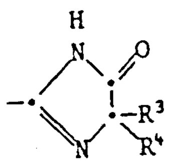

et

$R^3$ représente un alkyle en $C_1$-$C_4$ et

$R^4$ représente un reste méthyle ou éthyle substitué avec 1 à 5 atomes de fluor,

ainsi que les sels des composés de formule I avec les acides, les bases et les complexants.

**2.** Composés de formule I selon la revendication 1 où

X représente CH ou N,

$R^1$ et $R^2$ représentent, indépendamment l'un de l'autre, l'hydrogène ou un alkyle en $C_1$-$C_4$; ou

$R^1$ et $R^2$ forment ensemble avec les atomes de carbone du noyau hexagonal auxquels ils sont liés, un noyau carbocyclique pentagonal ou hexagonal insaturé ou en partie insaturé ou un noyau hétérocyclique pentagonal ou hexagonal contenant un atome de O ou de S et

A représente OH; $O^{\ominus}M^{\oplus}$; un alcoxy en $C_1$-$C_4$; un alcényle en $C_3$-$C_5$ oxy; un alcynyle en $C_3$-$C_5$ oxy; et

$M^{\oplus}$ un équivalent cation d'un métal alcalin ou alcalino-terreux; ou d'une base azotée; et

B représente une imidazolone de formule

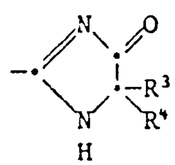

et

$R^3$ représente un alkyle en $C_1$-$C_4$; et

$R^4$ représente un reste méthyle ou éthyle substitué par 1 à 5 atomes de fluor.

**3.** L'acide 2-(5-méthyl-5-trifluorométhyl-imidazol-4-on-2-yl)-isonicotinique,
l'acide 2-(5-méthyl-5-trifluorométhyl-imidazol-4-on-2-yl)-5-méthyl-isonicotinique,
l'acide 2-(5-méthyl-5-trifluorométhyl-imidazol-4-on-2-yl)-5-éthyl-isonicotinique,
l'acide 2-[5-(1-fluoroéthyl)-5-méthyl-imidazol-4-on-2-yl]-isonicotinique,
l'acide 2-[5-(1-fluoroéthyl)-5-méthyl-imidazol-4-on-2-yl]-5-méthyl-isonicotinique,
l'acide 2-[5-méthyl-5-trifluorométhyl-imidazol-4-on-2-yl]-quinoléine-3-carboxylique ou
l'acide 2-[5-(1-fluoroéthyl)-5-méthyl-imidazol-4-on-2-yl]-5-éthyl-isonicotinique,
l'acide 2-[5-difluorométhyl-5-méthyl-imidazol-4-on-2-yl]-isonicotinique,
l'acide 2-[5-difluorométhyl-5-méthyl-imidazol-4-on-2-yl]-5-méthyl-isonicotinique,
l'acide 2-[5-difluorométhyl-5-méthyl-imidazol-4-on-2-yl]-5-éthyl-isonicotinique,
l'acide 2-[5-difluorométhyl-5-méthyl-imidazol-4-on-2-yl]-5,6-dihydro-cyclopenta[b]-pyridine-3-carboxylique,

l'acide 2-[5-difluorométhyl-5-méthyl-imidazol-4-on-2-yl]-5,6,7,8-tétrahydroquinoléine-3-carboxylique,
l'acide 2-[5-difluorométhyl-5-méthyl-imidazol-4-on-2-yl]-quinoléine-3-carboxylique,
l'acide 2-[5-(1-fluoroéthyl)-5-méthyl-imidazol-4-on-2-yl]-5,6-dihydro-cyclopenta[b]pyridine-3-carboxylique,
l'acide 2-[5-(1-fluoroéthyl)-5-méthyl-imidazol-4-on-2-yl]-5,6,7,8-tétrahydroquinoléine-3-carboxylique,
l'ester méthylique de l'acide 2-[5-(1-fluoroéthyl)-5-méthyl-imidazol-4-on-2-yl]isonicotinique,
l'ester éthylique de l'acide 2-[5-(1-fluoroéthyl)-5-méthyl-imidazol-4-on-2-yl]-isonicotinique ou
l'ester éthylique de l'acide 2-[5-(1-fluoroéthyl)-5-méthyl-imidazol-4-on-2-yl]-5-éthyl-isonicotinique selon la revendication 1 ou 2.

**4.** Procédé pour la préparation des composés de formule Ia

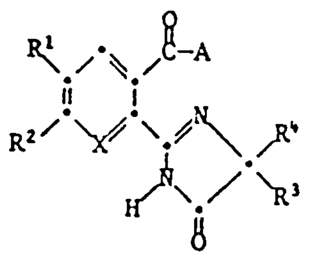

Ia

où les restes $R^1$ à $R^4$, X et A sont définis comme dans la revendication 1 ou 2, caractérisé en ce que l'on fait réagir un diester de formule IV

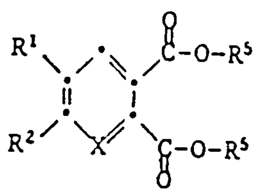

IV,

où X, $R^1$ et $R^2$ sont définis comme précédemment et les restes $R^5$ représentent, indépendamment l'un de l'autre, un alkyle en $C_1$-$C_6$ avec un amide d' $\alpha$-amino-acide de formule II

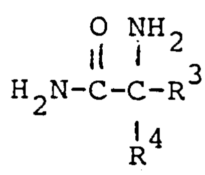

II

en présence d'une quantité au moins équimolaire d'une base et en ce que l'on traite ensuite le mélange réactionnel par hydrolyse.

**5.** Procédé pour la préparation des sels de formule Ia'

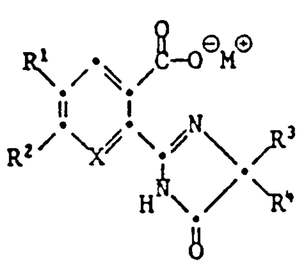

Ia'

selon la revendication 1 ou 2,
où X, $R^1$, $R^2$, $R^3$, et $R^4$ sont définis comme précédemment et où $M^{\oplus}$ représente un équivalent cation d'un métal alcalin ou alcalino-terreux ou une base azotée,
caractérisé

a) en ce que l'on fait réagir un composé de formule Ia

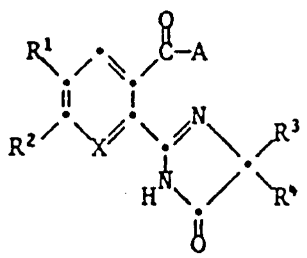

Ia,

dans laquelle les restes X, $R^1$, $R^2$, $R^3$ et $R^4$ sont définis comme précédemment et A représente OH avec une quantité équivalente d'un composé de formule VII

$OH^{\ominus}$ $M^{\oplus}$ VII

dans laquelle $M^{\oplus}$ représente un équivalent cation d'un métal alcalin ou alcalino-terreux ou une base azotée quaternaire ou

b) en ce que l'on fait réagir directement un composé de formule Ia dans laquelle A représente OH avec un métal alcalin ou alcalino-terreux ou avec un alcali ou un sel alcalino-terreux ou une base azotée.

**6.** Agent herbicide contenant un composé de formule I selon l'une des revendications 1 à 3 à côté d'autres adjuvants et/ou supports.

**7.** Procédé pour lutter contre la croissance des plantes indésirées, caractérisé en ce que l'on laisse agir un composé de formule I selon les revendications 1 à 3 ou un agent selon la revendication 6 à l'endroit où se trouve la plante à combattre.

**8.** Semences caractérisées par une teneur en un composé de formule I, efficace comme herbicide, selon l'une des revendications 1 à 3.

**9.** Amides d' $\alpha$ -amino-acides de formule II

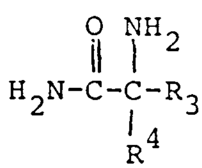

II,

où

$R^3$ représente un alkyle en $C_1$-$C_4$ et

$R^4$ représente un reste méthyle ou éthyle substitué par 1 à 5 atomes de fluor.

**10.** Procédé pour la préparation des amides d' $\alpha$ -amino-acides de formule II selon la revendication 9, caractérisé

a) en ce que l'on fait réagir une oxazolin-5-one de formule XI où $R^3$ et $R^4$ sont définis comme précédemment, avec $NH_3$ pour former le composé XII et en ce que l'on clive sur le composé XII les groupes protecteurs trifluoroacétyles,

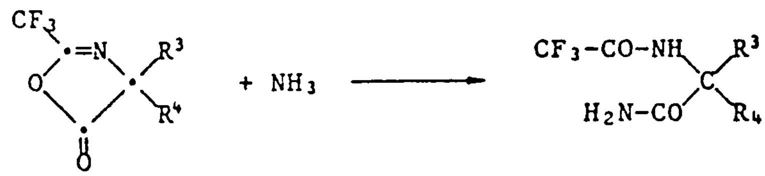

XII

XII 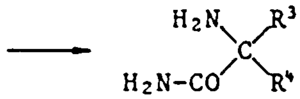

ou

b) en ce que l'on additionne une cétone XIII où les restes $R^3$ et $R^4$ sont définis comme précédemment, $NH_3$ et un cyanure dans les conditions de la synthèse de Strecker pour former un composé XIV et en ce que l'on hydrolyse exclusivement le composé XIV pour former le composé II

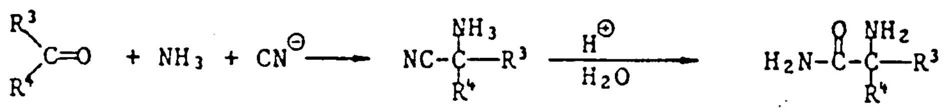


XIII XIV II

**Revendications pour l'Etat contractant suivant : ES**

**1.** Procédé pour la préparation des composés de formule Ia

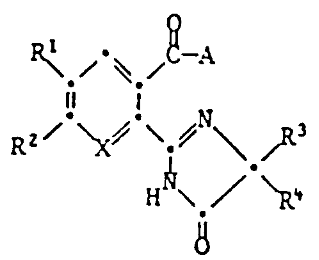

Ia,

où

| | |
|---|---|
| X | représente CH ou N, |
| $R^1$ et $R^2$ | représentent, indépendamment l'un de l'autre, l'hydrogène; un alkyle en $C_1$-$C_4$ éventuellement substitué une ou plusieurs fois par un halogène, par le nitro, par le cyano, par un alcoxy en $C_1$-$C_4$, par un halogénoalcoxy en $C_1$-$C_4$, par un alkyle en $C_1$-$C_4$ thio ou par un halogénoalkyle en $C_1$-$C_4$ thio ou |
| $R^1$ et $R^2$ | forment ensemble avec les atomes de carbone du noyau hexagonal auxquels ils sont liés un noyau carbocyclique pentagonal ou hexagonal insaturé ou en partie insaturé ou un noyau hétérocyclique pentagonal ou hexagonal contenant jusqu'à deux atomes de O ou de S et |
| A | représente OH; $O^-M^+$ ; un alcoxy en $C_1$-$C_4$, un alcényle en $C_3$-$C_5$ oxy, un alcynyle en $C_3$-$C_5$ oxy ou un alcoxy en $C_1$-$C_4$ alcoxy en $C_1$-$C_4$; et |
| $M^+$ | un équivalent cation d'un métal alcalin ou alcalino-terreux ou d'une base azotée et |
| $R^3$ | représente un alkyle en $C_1$-$C_4$ et |
| $R^4$ | représente un reste méthyle ou éthyle substitué par 1 à 5 atomes de fluor |

caractérisé en ce que l'on fait réagir un diester de formule IV

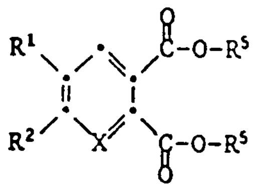 IV,

où X, $R^1$ et $R^2$ sont définis comme précédemment et les restes $R^5$ représentent, indépendamment l'un de l'autre, un alkyle en $C_1$-$C_6$ avec un amide d' $\alpha$ -amino-acide de formule II

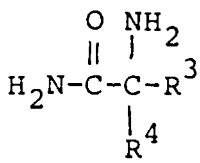 II

en présence d'une quantité au moins équimolaire d'une base et en ce que l'on traite ensuite le mélange réactionnel par hydrolyse.

**2.** Procédé pour la préparation des sels de formule Ia'

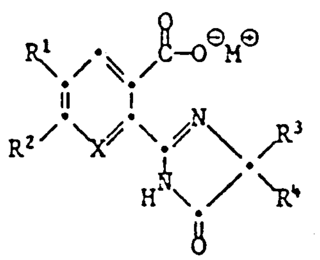 Ia'

où X, $R^1$, $R^2$, $R^3$ et $R^4$ sont définis comme dans la revendication 1 et $M^{\oplus}$ représente un équivalent cation d'un métal alcalin ou alcalino-terreux ou d'une base azotée,
caractérisé

a) en ce que l'on fait réagir un composé de formule Ia

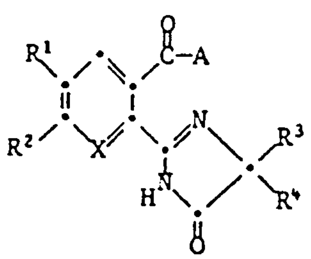 Ia,

dans laquelle les restes X, $R^1$, $R^2$, $R^3$ et $R^4$ sont définis comme précédemment et A représente OH avec une quantité équivalente d'un composé de formule VII

$OH^{\ominus}$ $M^{\oplus}$ VII

dans laquelle $M^{\oplus}$ représente un équivalent cation d'un métal alcalin ou alcalino-terreux ou une base azotée quaternaire ou

b) en ce que l'on fait réagir directement un composé de formule Ia dans laquelle A représente OH

avec un métal alcalin ou alcalino-terreux ou avec un alcali ou un sel alcalino-terreux ou une base azotée.

**3.** Procédé pour lutter contre la croissance des plantes indésirées, caractérisé en ce que l'on laisse agir un composé de formule I

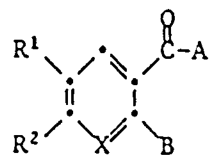

I,

où

X représente CH ou N,

$R^1$ et $R^2$ représentent, indépendamment l'un de l'autre, l'hydrogène; un alkyle en $C_1$-$C_4$ éventuellement substitué une ou plusieurs fois par un halogène, par le nitro, par le cyano, par un alcoxy en $C_1$-$C_4$, par un halogénoalcoxy en $C_1$-$C_4$, par un alkyle en $C_1$-$C_4$ thio ou par un halogénoalkyle en $C_1$-$C_4$ thio ou

$R^1$ et $R^2$ forment ensemble avec les atomes de carbone du noyau hexagonal auxquels ils sont liés un noyau carbocyclique pentagonal ou hexagonal insaturé ou en partie insaturé ou un noyau hétérocyclique pentagonal ou hexagonal contenant jusqu'à deux atomes de O ou de S et

A représente OH; $O^-M^+$ ; un alcoxy en $C_1$-$C_4$, un alcényle en $C_3$-$C_5$ oxy, un alcynyle en $C_3$-$C_5$ oxy ou un alcoxy en $C_1$-$C_4$ alcoxy en $C_1$-$C_4$ et

$M^+$ un équivalent cation d'un métal alcalin ou alcalino-terreux ou d'une base azotée et

B une imidazole de formule

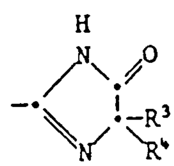

et

$R^3$ représente un alkyle en $C_1$-$C_4$ et

$R^4$ représente un reste méthyle ou éthyle substitué avec 1 à 5 atomes de fluor,

à l'endroit où se trouvent les plantes à combattre.

**4.** Procédé selon la revendication 3 pour lutter contre les mauvaises herbes ou graminées des cultures de plantes utiles.

**5.** Procédé pour la préparation d'un agent herbicide, caractérisé en ce que l'on mélange un composé de formule I

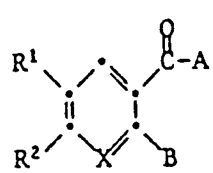

I,

où les restes $R^1$, $R^2$, X, A et B sont définis comme dans la revendication 3, avec des adjuvants et/ou supports.

**6.** Procédé pour le traitement des semences, caractérisé en ce que l'on applique une quantité efficace en tant qu'herbicide d'un composé de formule I

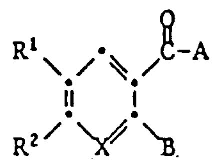

I,

où les restes $R^1$, $R^2$, X, A et B sont définis comme dans la revendication 3 sur les semences.

**7.** Procédé pour la préparation des amides d' α-amino-acides de formule II

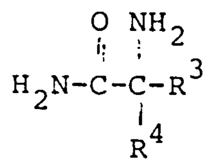

II

où

$R^3$ représente un alkyle en $C_1$-$C_4$ et

$R^4$ représente un reste méthyle ou éthyle substitué par 1 à 5 atomes de fluor,

caractérisé

a) en ce que l'on fait réagir une oxazolin-5-one de formule XI où $R^3$ et $R^4$ sont définis comme précédemment, avec $NH_3$ pour former le composé XII et en ce que l'on clive sur le composé XII les groupes protecteurs trifluoroacétyles.

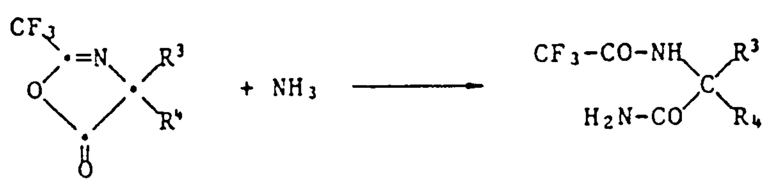

XII

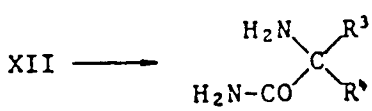

ou

b) en ce que l'on additionne une cétone XIII où les restes $R^3$ et $R^4$ sont définis comme précédemment, $NH_3$ et un cyanure dans les conditions de la synthèse de Strecker pour former un composé XIV et en ce que l'on hydrolyse exclusivement le composé XIV pour former le composé II

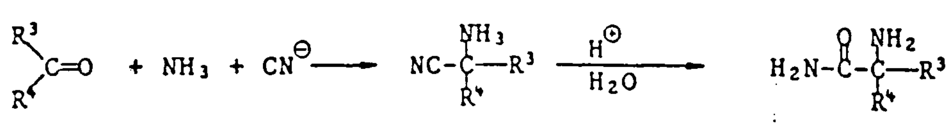

XIII XIV II